# EUROPEAN PATENT APPLICATION

(11) **EP 4 806 907 A1**
(43) Date of publication of application: **16.09.2026**
(21) Application number: 24887176.6
(22) Date of filing: 06.11.2024
(51) Int. Cl.: C07K 14/33, C12N 15/31, C12N 15/63, A61K 39/08, A61P 31/04, C12R 1/145

(54) **MOLECULE OF RECOMBINANT NUCLEIC ACID, EPITOPE OF ALPHA TOXIN OF RECOMBINANT C. NOVYI AND USE THEREOF, EXPRESSION VECTOR, VACCINE COMPOSITION, METHOD FOR PREVENTING OR TREATING CLOSTRIDIOSIS IN AN INDIVIDUAL SUFFERING FROM SAME AND METHOD FOR PRODUCING A VACCINE COMPOSITION AGAINST CLOSTRIDIOSIS**

(30) Priority: 08.11.2023 BR 102023023411
(71) Applicant: Fundação Oswaldo Cruz, 21040-360 Rio de Janeiro, RJ (BR)
(72) Inventor: MARIUBA, Luis André Morais, 69057-070 Manaus-AM (BR); NOGUEIRA, Paulo Afonso, 69019-618 Manaus-AM (BR); ALMEIDA, Maria Edilene Martins De, 69028-302 Manaus-AM (BR); SILVA, Elizangela Farias Da Silva, 69093-493 Manaus-AM (BR); SOARES, Andreimar Martins, 76820-374 PORTO VELHO-RO (BR); LIMA, Anderson Maciel De, 69020-020 MANAUS-AM (BR); CANGUSSU, Alex Sander Rodrigues, 77413-105 GURUPI-TO (BR); DEUSDARÁ, Tullio Teixeria, 77415-470 GURUPI-TO (BR); FÉLIX, Mellanie Karoline Do Carmo, 77410-460 GURUPI-TO (BR); ASTOLFI FILHO, Spartaco, 69008-310 MANAUS-AM (BR); CANGUSSU, Keoma Dias Pires, 77413-105 GURUPI-TO (BR); SILVA, Raquel Stefanni Rodrigues Da, 69033-100 MANAUS-AM (BR); GLÓRIA, Juliane Corrêa, 69057-250 MANAUS-AM (BR); MELO, Joyce Marinho, 69063-690 MANAUS-AM (BR); SILVA, Eliza Raquel Duarte Da, 69092-564 MANAUS-AM (BR); BRAGA, Darleide Dos Santos, 69088-024 MANAUS-AM (BR)
(74) Representative: Dragotti & Associati S.P.A.
(86) International application number: PCT/BR2024/050509
(87) International publication number: WO 2025/097230

(57) **Abstract**

The present invention provides recombinant nucleic acid molecules encoding recombinant alpha toxin epitopes of *C. novyi.* Additionally, the invention provides vaccine compositions comprising such epitopes and the use thereof, and method for producing such compositions. Finally, the invention also provides method of treating and preventing clostridiosis.

## Description

### FIELD OF THE INVENTION

The present invention relates to the field of veterinary medicine and biotechnology. Specifically, the present invention relates to the development and production of epitopes from *C. novyi* toxin in the culture scaling process in *Escherichia coli* BL21 (DE3) and use thereof in vaccine formulations for clostridiosis in bovines.

### BACKGOUND OF THE INVENTION

Cattle farming is one of the most important economic activities in Brazil. The Brazilian Institute of Geography and Statistics (in Portuguese, *Instituto Brasileiro de Geografia e Estatística - IBGE*) estimates that the country currently has more than 220 million head of cattle, with the Central-West region having the largest number of these livestock (IBGE, 2021). With the professionalization of cattle farming, disease control and product quality monitoring have become increasingly stringent (Ferraz and Felício, 2010; Carvalho and Zen, 2017).

There is a group of infections and intoxications that affect herds and are caused by anaerobic bacteria of the genus *Clostridium*, known as clostridiosis. Depending on the type of bacteria from the genus *Clostridium*, the following may occur: pulpy kidney disease, hemorrhagic enterotoxemia, necrotizing infectious hepatitis, and sudden death (Embrapa, 2001).

The clostridiosis are typical diseases that affect these herds, caused by bacteria of the genus *Clostridium*, which are characterized by being anaerobic, gram-positive, motile rods and by causing significant losses in cattle farming (Ma et al., 2007; Lobato et al., 2013; Walker et al., 2016).

Strains of this genus cause disease through the presence of toxins and / or by invading tissues, differentiating according to the presence and synthesis of genes (Uzal et al., 2010; Brandi et al, 2014; Brandi et al., 2016; Fouad et al., 2021).

The *C. novyi* is a common agent in domestic animals and humans and is divided into four types based on the production of toxins: A, B, C and D, type D being recognized as *C. haemolyticum*. These potent exotoxins mediate clostridial pathogenesis (Uzal et al., 2010; Navarro et al., 2017).

*C. novyi* type B produces alpha toxin, the main cause of the pathogenesis of this strain (Le Gratiet et al., 2021). This toxin belongs to the group of large clostridial toxins, with a molecular mass of 250 kDa (Lima et al., 2011), with glycosyltransferase action, through which it causes the inactivation of proteins present in the cytoskeleton and disorganization of actin filaments, resulting in cell rounding, loss of intercellular junctions, and increased endothelial permeability (Gonçalves et al., 2013).

The *alpha toxin from C. novyi* is produced by types A and B, coded by the chromosomal gene *cpa* or *plc*; while beta toxin is produced by types B and D, coded by the genes contained in plasmids. Alpha toxin is lethal; beta toxin, classified as necrotizing and hemolytic, is generally not produced in lethal quantities (Navarro et al., 2017).

The alpha toxin or PLC is part of the group of the great clostridial toxins, with molecular weight greater than 250 kDa. The action of this class causes cell rounding, an effect that alters the cytoskeleton of mammalian cells (Lima et al., 2020).

The alpha toxin produced is defined as a glycosyltransferase is responsible for catalyzing the glycosylation of small GTPases, causing the inactivation of proteins present in the cytoskeleton and disorganization of actin filaments, resulting in cell rounding, loss of intercellular junctions, and increased endothelial permeability (Lobato et al., 2013, Lobato et al., 2021).

Studies of the three-dimensional structure of the alpha toxin show two main domains: the N-terminal (amino-terminal) domain, featuring single-domain proteins and the C-terminal (carboxy-terminal) domain, featuring proteins with two domains (Noshahri et al., 2016). The N-terminal domain of alpha toxin retains phospholipase activity and exhibits loss of hemolytic activity. The C-terminal domain influences the enzymatic activity of the N-terminal domain and recognizes membrane phospholipids, being extremely important for the hemolytic activity of alpha toxin (Nagahama et al., 2013).

That is, the alpha toxin has a tripartite structure in which enzymatic activity is related to the N-terminal region, known as the catalytic domain; the binding to the receptor is related to the C-terminal region of the toxin; and the translocation of the toxin to the cytosol is related to the hydrophobic domain in the central region (Nagahama et al., 2013; Noshahri et al., 2016).

Liver necrosis is one of the diseases resulting from infection caused by *C. novyi* type B (Ma et al., 2007). This disease is associated with trematodes, such as Fasciola hepatica. However, there are indications of the disease in the absence of these parasites (Lobato et al., 2008; Lobato et al., 2013).

In its structure, the alpha toxin features zinc metals strongly bonded to an interchangeable divalent cation. His-68, -126 and -136 residues bind to a divalent cation that is necessary for binding to membranes. His-148 and Glu-152 bind to a zinc ion that is essential for the toxin's active site and His-11 and Asp-130 bind firmly to the other zinc ion, which is needed for maintaining the structure.

Amino acids Tyr-57 and -65 play roles in the penetration of the toxin into the membrane bilayer and also allow access to the sphingomyelin catalytic site (Nagahama et al., 2006; Nagahama et al., 2013; Oda et al., 2015).

The hemolytic, myotoxic, and cytotoxic activity of the alpha toxin can be a consequence of their interaction with the membranes of host cells and the subsequent hydrolysis of phospholipids (Alape-Girón et al., 2000). Furthermore, reports describe the action of the alpha toxin with phospholipase C (PLC) activity and sphingomyelinase (SMase) activity.

Recent findings have revealed that a membrane localization domain (MLD) has been identified as a conserved domain among numerous bacterial toxins, including the large family of clostridial glycosylating toxins (LCGTs), which plays key roles in plasma membrane localization (Liu et al., 2022).

As already disclosed herein, the alpha toxin or PLC, of *C. novyi* type B, belongs to the LCGT glycosylating group with phospholipase C and sphingomyelinase activity, with a C-terminal region directly related to binding to a membrane receptor that recognizes membrane phospholipids (Noshahri et al., 2016; Liu et al., 2022).

Besides, in several *Clostridium* strains, the *plc* gene is located at the same locus, near the putative origin of replication and between two ribosomal RNA operons. It is worth noting that this is a region of high relevance, since this region of the chromosome is not only the first to be replicated, but also the most conserved segment of the genome containing *housekeeping* genes (constitutive genes essential for the existence of a cell, and expressed in all cells of an organism under normal and pathophysiological conditions).

Reports have already described that the nucleotide sequence of the *plc* gene is 2.2 kb, revealing an open reading frame which comprises amino acids from 943 to 2137, corresponding to a protein of molecular weight of 43.3 kDa. The following amino acid sequences encoded between the initiation codon and nucleotide 1027 form a signal sequence for the direct transport of alpha toxin across the cell membrane (Tsutsui et al., 1995; Noshahri et al., 2016; Liu et al., 2022).

Another relevant aspect is that the alpha toxin can exhibit two distinct conformations: an open form containing the accessible active site and a closed form with the active site covered, encompassing residues 135 to 150. Predictions of the structure of alpha toxin revealed that the N-terminal region contains 687 residues of amino acids and is located in the first protein portion and is smaller than the C-terminal portion.

On the other side, the C-terminal region of the protein consisting of approximately 1000 residues of amino acids is more antigenic, given the greater presence of beta-sheet structures, resulting in a stronger immune response compared to helical regions (Noshahri et al., 2016).

Furthermore, the binding of the C-terminal domain to the target membrane causes the active site to be positioned towards the lipid bilayer, leading to its exposure, a fact that makes it important for the enzyme's toxicity (Walker et al., 2000; Alape-Girón et al., 2000; Oda, et al., 2015).

Other studies have already described that when animals are immunized using the C-terminal domain, the generated antibody production ensures protection after challenge with a lethal dose of active toxin, revealing it to be an important, highly antigenic region with a high probability of humoral immune response.

On the other hand, regions of the N-terminal domain exhibit a high potential for binding to the histocompatibility complex II (MHCII) (Nagahama et al., 2013).

Infection occurs through latent spores that germinate in the liver, occurring in areas damaged by the migration of *Fasciola hepatica*. The spores develop into a vegetative form and trigger the production of large quantities of alpha toxin (Uzal, 2003; Lima et al., 2011).

Systemic effects cause the animal's death (Farias et al., 2014; Navarro et al., 2017). The multiplication of *C. novyi* type B in the liver can also cause sudden death (Lobato et al., 2008; Aquino et al., 2016; Radostits et al., 2002).

Some characteristics of the causative agent of clostridiosis make its eradication difficult since these bacteria are capable of forming resistant spores and are ubiquitous in the gastrointestinal tract of animals and humans.

For this reason, control is mainly based on preventive measures and systematic vaccination of animals (Lima et al., 2011; Skarin; Segerman, 2014).

However, for some types of etiological agents of the genus *Clostridium*, such as *Clostridium novyi* type B, there are still technical barriers to overcome, such as the immunogenic properties of vaccine formulations (Nascimento et al. 2004).

### Vaccines used to control hepatic necrosis

The veterinary pharmaceutical market has been showing growth due to demand from the production animal segment, particularly livestock farming (Ferraz and Felício, 2010; Carvalho and Zen, 2017).

Immunization of these animals against clostridiosis is performed using concentrated toxoids, in which two doses of vaccine can confer immunity for up to one year; however, there is no official control to verify the vaccine efficacy of these immunogens (Lobato et al., 2008; Lima et al., 2011; Lobato et al., 2013).

This market is dominated by multinational companies whose clostridial vaccines contain inactivated toxins in their formulation, with multiple antigens and a wide variety of agents and toxins. The vast majority are composed of antigens for myonecrosis, enterotoxemia, bacillary hemoglobinuria, hepatic necrosis, botulism, and tetanus (Lobato et al., 2013).

In their study, Nascimento et al. (2004) analyzed vaccines against *C. novyi* type B and demonstrated low immunogenic power of the products sold in the national market.

Because it is a fastidious microorganism, obtaining *alpha toxin from C. novyi* has been costly, leading to instances of failure during stages of industrial bioprocesses (Zeng et al., 2011; Aquino et al., 2016; Navarro et al., 2017).

Technological alternatives, such as recombinant protein expression, have been the subject of *in silico* studies for the selection of vaccine epitope regions (Lobato et al., 2013; Lima et al., 2020; Le Gratiet et al., 2021).

Research related to the development of vaccines formulated with recombinant protein, obtained from the fermentation of genetically modified microorganisms, reduce production costs and provide standardization of production. Analyses of hydrophobicity, accessibility, antigenicity, and protein flexibility can be performed using bioinformatics tools, enabling the selection of potential regions for protein construction (Santos et al., 2012). Along these lines, a relevant aspect is the use of epitopes from the conserved gene region of *C. novyi* in vaccine formulations. Patent CN110041437-B uses the C fragments of the tetanus toxin and three N-terminal epitopes and one C-terminal epitope of *Clostridium novyi* alpha toxin, however, do not use genes from a conserved C-terminal region.

However, recombinant vaccines developed from antigens originating from conserved regions allow for greater immune stimulation of the host, since these regions are always present in bacterial infections, and which allows them to be described as primary targets in the development of recombinant vaccines. Furthermore, these regions offer greater protection compared to stimuli induced by bacterin-based vaccines, since the latter generates limited protection (Perez-Casal, J. et al., 2017; Baruah, N. et al.,2021; Cangussu, A.S.R. et al., 2018).

The production of recombinant *alpha toxin from C. novyi* has been commonly obtained by transforming *Escherichia coli* BL 21 (Zeng et al., 2011).

### Expression of recombinant proteins

In silico studies allow the obtaining of selectable epitopes that are employed in the construction of expression vectors (Sobrinho et al., 2010; Santos et al., 2012; Santos et al., 2019; Brito et al., 2021), being validated after synthesis in cell cultures, as well as by reactivity with sera from infected animals (Zeng et al., 2011; Silvestre et al., 2014; Moreira et al., 2016; Cangussu et al., 2018; Félix et al., 2019; Félix et al., 2022).

The production and expression of recombinant proteins use different host cells into which these regions of interest are introduced. Through recombinant DNA technology, it is possible to obtain pure and functional proteins at low cost (Lee et al., 2003; Nosshahri et al., 2016; Kojima et al., 2022).

Several expression systems are employed for this purpose, with the enterobacterium *E. coli* being the most commonly used, with records showing up to 60 % utilization for the production of recombinant proteins (Duarte et al., 2021; Cardoso et al., 2022). Their choice is justified by the large volume of genetic, physiological, biochemical information and tools available for their genetic manipulation (Correa and Oppezzo, 2011; Zeng et al., 2011).

Several vectors are available for protein expression in *E. coli*, promoting high levels of expression, stability, and versatility (Jonasson et al., 2002). In addition to these factors, the efficiency of clostridial vaccines is related to the adjuvants that compose them (Assis et al., 2002).

### Oily adjuvant and microemulsion

The adjuvant is one of the most principal factors in vaccine composition and is directly linked to increasing the immunogenicity of vaccines. Veterinary vaccines most often use adjuvants based on aluminum and mineral oil (Park et al., 2014; Veenstra et al., 2017).

Aluminum-based adjuvants have several disadvantages, such as inducing short-lived antibody responses that lead to frequent revaccinations and side effects. In contrast, oil-based adjuvants have the advantage of inducing high antibody titers and long-lasting antibody responses that lead to effective protection (Khorasani et al., 2016; Tehrani et al., 2016).

Emulsified oil adjuvants influence the antigen in terms of time, location, and concentration through the "depot effect." The microemulsion forms a semisolid mass at the injection site, slowly releasing the antigen into the tissue or bloodstream, increasing, and prolonging the humoral response (Tehrani et al., 2016; Veenstra et al., 2017).

These systems have the advantage of good injection capacity due to their low oil content and viscosity, and can easily present the antigen (Leclercq et al., 2011; Sun et al., 2015).

Antigens with emulsified adjuvants initiate the innate immune response, which plays a role in activating the adaptive immune response by stimulating specific components of the humoral or cell-mediated immune response (Grun and Maurer, 1989; Veenstra et al., 2017).

Given this context and considering the relevance of these immunogens present in vaccine formulations against clostridiosis, the present invention proposes the optimization and scaling of obtaining *alpha toxin from C. novyi* recombinant epitopes in recombinant *E. coli* BL21 pLysS (DE3) culture.

In this regard, three different combinations of epitopes (DE3 / Ep1, DE3 / Ep2 and DE3 / Ep1+DE3 / Ep2) of recombinant *alpha toxin from C. novyi* type B in W/O emulsion were proposed. Furthermore, the immunogenicity and protection parameters of immunized and challenged murine models were determined, aiming at new alternatives and advances for antigen adsorption applicable to the control of clostridiosis. In the epitope selection process, genetic engineering tools were used, in which the DE3 / Ep1 epitope comprises the N-terminal domain region (between the amino acids 1 and 583, vaccine group G2), epitope DE3 / Ep2 comprises the C-terminal domain region (between the amino acids 1599 and 2178, vaccine group G3), combined epitopes DE3 / Ep1 + DE3 / Ep2 comprised both domains (vaccine group G4). Groups G2, G3 and G4 were compared with the group G1 (adjuvant MONTANIDE^{™} ISA 61 VG, Seppic, Brazil) and showed better vaccine efficacy data.

With this, a vaccine formulation with two epitopes was developed from the sequence of the *alpha toxin from C. novyi,* GenBank CAA88565.1, located in the amino acid 491° to 583° of the N-terminal region, and amino acids 1599° to 1779° of the C-terminal region. The formulation process was prepared as a water-in-oil emulsion in a 40 / 60 ratio, where *in vivo* tests were performed and yielded results indicating potential as an immunogenic composition in combating the group of diseases caused by the agent *C. novyi* type B.

The results indicate effectiveness in its use as an immunogen in vaccine protection, as well as protection against damage caused by the active *alpha toxin from C. novyi* type B. The experimental group G3 (C-terminal) showed a higher vaccine efficacy rate (40 %) when compared to group G2 (N-terminal), with 25 %. When the domains were combined (G4), immunization of mice produced a synergistic effect on protective potency, with 75 % of animals surviving after a lethal challenge dose with the active toxin.

Finally, A mechanically stirred tank biological reactor was employed to promote improvements on the bioprocess conditions and enable proposals for scaling up production of alpha toxin epitopes of *C. novyi* in order to contribute to competitive gains for the veterinary vaccine manufacturing industry.

### SUMMARY OF THE INVENTION

The present invention has the aim to provide alpha toxin epitopes of *C. novyi* for the production of vaccine composition, as well as the method for producing the same. Preferably, the epitopes originate in the conserved region of the *C. novyi* gene.

In a first embodiment, the present invention provides recombinant nucleic acid molecules which comprise the nucleic acid sequences as defined by SEQ ID NO: 1 or by SEQ ID NO: 3 and its degenerated sequences encoding the same polypeptidic sequence.

In a second embodiment, the present invention provides recombinant alpha toxin epitopes of *C. novyi* which comprise the peptide sequences as defined by SEQ ID NO: 2 or by SEQ ID NO: 4.

In a third embodiment, the recombinant alpha toxin epitopes of *C. novyi* further comprise a poly histidine tail (6xHIS) at the amino-terminal end.

In a fourth embodiment, the present invention provides expression vectors which comprise the nucleic acid molecules according to the present invention and their regulatory regions.

In a fifth embodiment, the expression vectors comprise the regulatory regions selected from BamHI and EcoRI.

In a sixth embodiment, the present invention provides vaccine compositions which comprise one or more epitopes according to the present invention and veterinary acceptable excipients.

In a seventh embodiment, the vaccine compositions comprise both epitopes according to the present invention and veterinary acceptable excipients.

In an eighth embodiment, the vaccine compositions can be of the water / oil emulsion type and are to prevent or treat clostridiosis in a subject suffering therefrom.

In a nineth embodiment, the present invention provides the use of an epitope as disclosed in the present application in the preparation of a vaccine composition to prevent or treat clostridiosis in a subject suffering therefrom.

In a tenth embodiment, the present invention provides a method for preventing or treating clostridiosis in a subject suffering therefrom comprising administering the vaccine composition as disclosed in the present application.

In an eleventh embodiment, the present invention provides a method for producing a vaccine composition against clostridiosis which comprises the following steps:
i) Transforming *E. coli* cells with an expression plasmid comprising the molecules as previously defined;
ii) Culturing said cells in bioreactors via the fed-batch process;
iii) Purifying the product of the culturing step ii);
iv) Preparing the vaccine composition comprising the epitopes obtained after step iii).

### BRIEF DESCRIPTION OF THE DRAWINGS

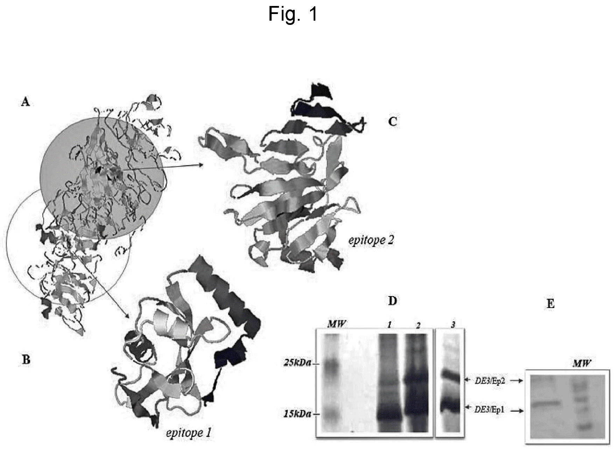

Figure 1: Figure 1 refers to *in silico* modeling and detection of vaccine epitopes. (A) 3D structure of the fraction of the *alpha toxin from C. novyi* type B predicted by i-TASSER from GenBank data: CAA88565.1 [48]. (B) and (C) 3D structure of DE3 / Ep1 and DE3 / Ep2, respectively. (D) Detection of DE3 / Ep1 (17kDa) and DE3 / Ep2 (26kDa) using 12 % gel electrophoresis. (E) *Western blotting* of vaccine epitope reactivity with serum from Swiss mice infected with wildtype *alpha toxin from C. novyi*.

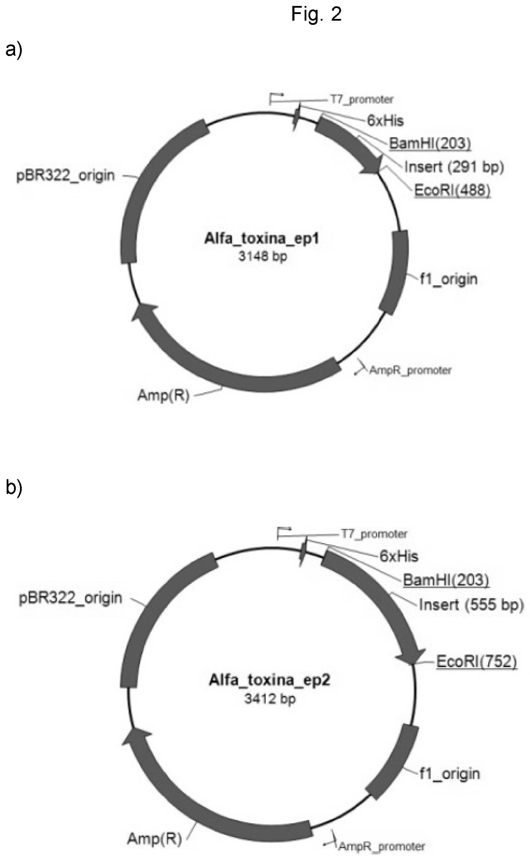

Figure 2: Figure 2 refers to the alpha toxin vectors ep1 and ep2 used by the present invention.

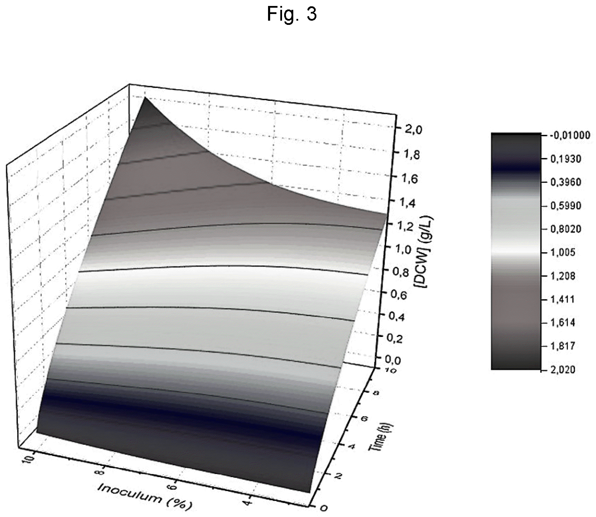

Figure 3: Figure 3 refers to surface response of DE3 cultivation in a rotary shaker and DCW ratio, inoculum fraction as a function of culture time (h) using response equations and analysis of variations by software Origin Pro^{®} 8.5. Data are represented by mean and standard deviation, considering the p-value (P < 0.05).

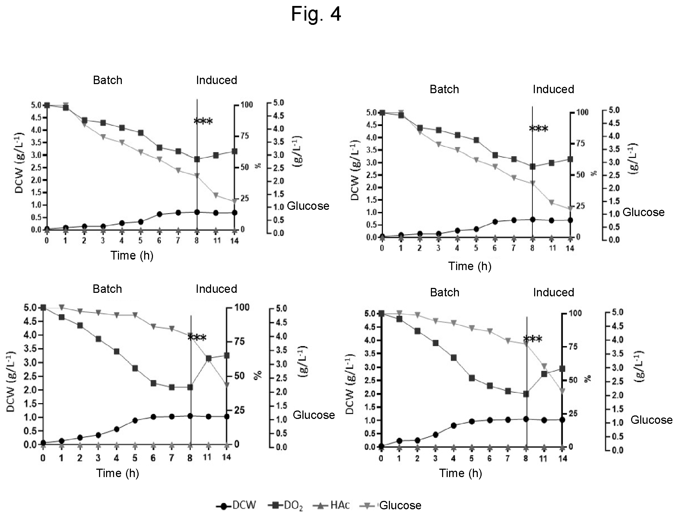

Figure 4: Figure 4 refers to the optimization of the production of vaccine epitopes in a stirred tank bioreactor. (A) and (B) Kinetic profiles of DE3 aiming at synthesis of epitopes DE3 / Ep1 and DE3 / Ep2 in simple batch. (C) and (D) Kinetic profile of DE3 aiming at the synthesis of DE3 / Ep1 and DE3 / Ep2 in fed-batch. Data obtained from DE3 cultures in SD medium containing ampicillin (100 µg / mL) conducted in a benchtop bioreactor (Tecnal BIO-TEC^{®}), at 37 °C. Glucose supplementation (500g / L) occurs at 30-minute intervals until maximum DCW. Induction conducted at 28 °C for 6 hours using lactose (10 g / L) as inductor. DCW - dry cell weight (g / L), O2 - dissolved oxygen (%), Hac - acetic acid concentration (g / L) and glucose (g / L). (*) beginning of induction, (→) beginning of feeding. Data is represented by mean and standard deviation, considering a p-value (p < 0.05) using software Origin Pro^{®} 8.5.

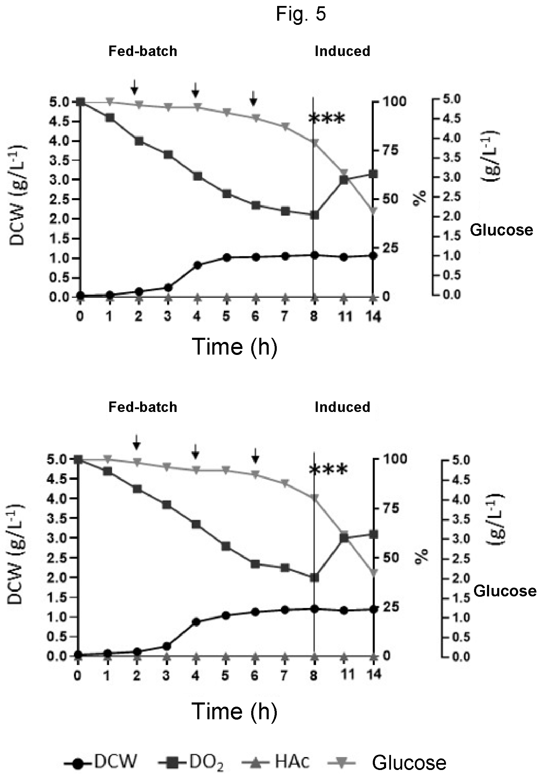

Figure 5: Figure 5 refers to the scaling up of the production of vaccinal epitopes in a non-stirred tank biological bioreactor. (A) and (B) Kinetic profiles of DE3 aiming at synthesis of epitopes DE3 / Ep1 and DE3 / Ep2. Data obtained from DE3 cultures in SD medium containing ampicillin (100 µg / mL) conducted in a Tecnal BIO-TEC^{®} bioreactor with an adapted fermentation tank using Thermo Scientific Nalgene^{®} round bottles at 37 °C. Glucose supplementation (500 g / L) occurs at 30-minute intervals until maximum DCW. Induction conducted at 28 °C for 6 h using a combination of lactose (10 g / L) and IPTG (0.2 mM) as inductor. DCW - dry cell weight (g / L), O2 - dissolved oxygen (%), Hac - acetic acid concentration (g / L) and glucose (g / L). (*) beginning of induction, (→) beginning of feeding. Data is represented by mean and standard deviation, considering a p-value (p < 0.05) using software Origin Pro^{®} 8.5.

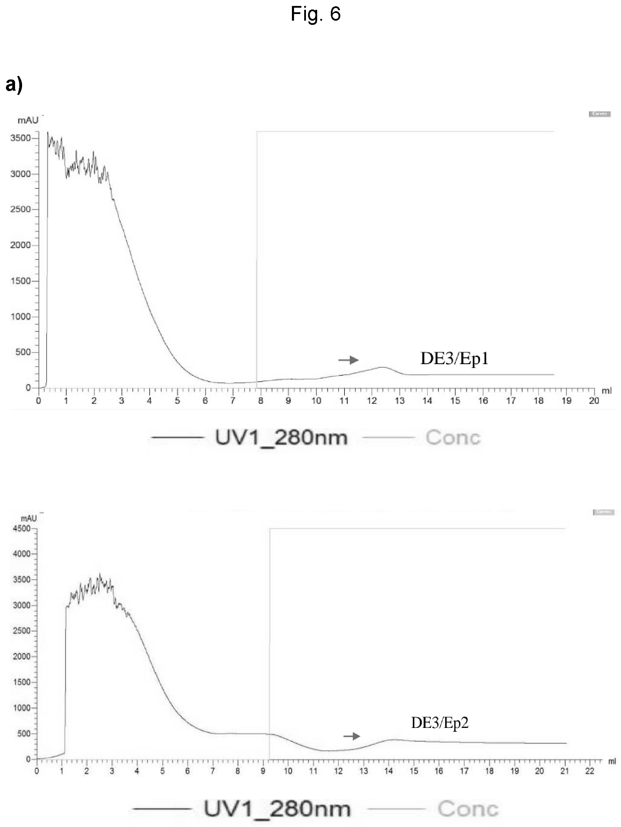

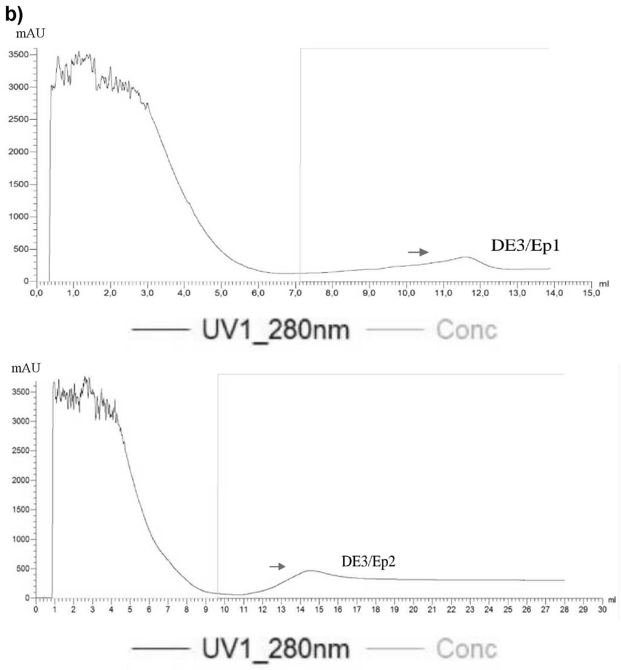

Figure 6: Figure 6 refers to the purification of vaccine epitopes in stirred tank biological bioreactor in lab scale. (A) Profiles of purified epitopes DE3 / Ep1 and DE3 / Ep2 obtained by simple batch. (B) Profiles of purified epitopes DE3 / Ep1 and DE3 / Ep2 obtained by fed batch. Data obtained from a Akta Purifier 10^{®} chromatography system, GE Lifescience Healthcare^{®}). Elutions determined at 280 nm.

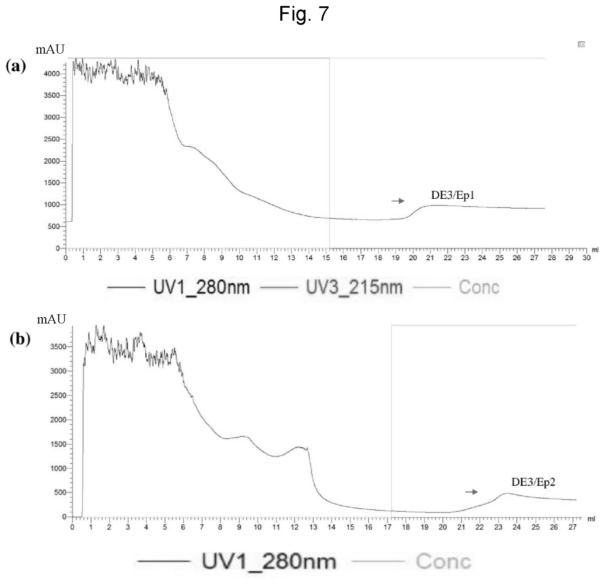

Figure 7: Figure 7 refers to the purification of vaccine epitopes in non-stirred tank biological bioreactor in scale-up. (A) and (B) Profiles of purified epitopes DE3 / Ep1 and DE3 / Ep2 obtained by fed batch. Data obtained from a Akta Purifier 10^{®} chromatography system, GE Lifescience Healthcare^{®}). Elutions determined at 280 nm.

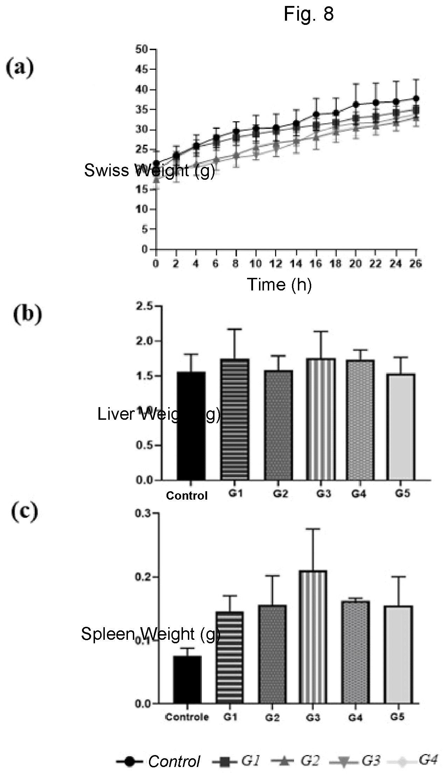

Figure 8: Figure 8 refers to Monitoring body weight of mice in experimental groups (A). Liver and spleen weight data (B and C). Control (non-immunized animals); G1 (animals inoculated with adjuvant in W/O microemulsion); G2 (immunized animals DE3 / Ep1 ALFA-T / REC in W/O microemulsion); G3 (immunized animals DE3 / Ep2 ALFA-T / REC in W/O microemulsion); G4 (immunized animals DE3 / Ep1 + DE3 / Ep2 ALFA-T / REC associated in W/O microemulsion); G5 (immunized animals with inactivated wildtype alpha toxin). P value p < 0.05 (*).

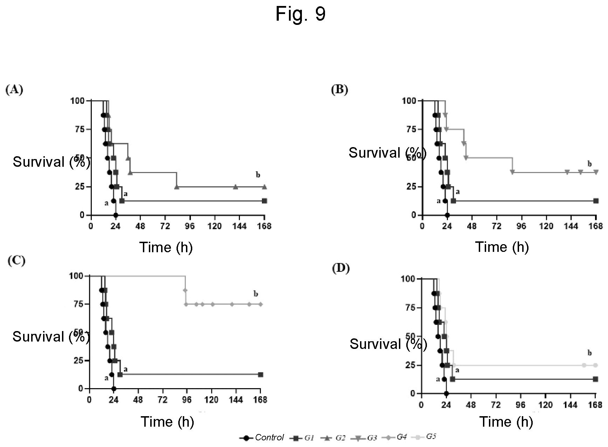

Figure 9: Figure 9 refers to the vaccine efficacy test using ALFA-T / REC vaccine epitopes with mice challenged with a lethal dose of active alpha toxin 157 DL50.g⁻¹. Control (non-immunized animals). (G1) Animals inoculated with adjuvant in microemulsion. (A) (G2) Survival curve of immunized mice ALFA-T / REC DE3 / Ep1 in W/O microemulsion. (B) (G3) Survival curve of immunized mice ALFA-T / REC DE3 / Ep2 in W/O microemulsion. (C) (G4) Survival curve of immunized mice ALFA-T / REC DE3 / Ep1 + ALFA-T / REC DE3 / Ep2 in combination and W/O microemulsion. (D) (G5) Survival curve of mice inoculated with inactivated alpha toxin after being challenged with a lethal dose of active alpha toxin 157 DL50.g⁻¹. *P* value determined by the log-rank test (Mantel-Cox). (*) p < 0.05 represents comparative analysis between groups.

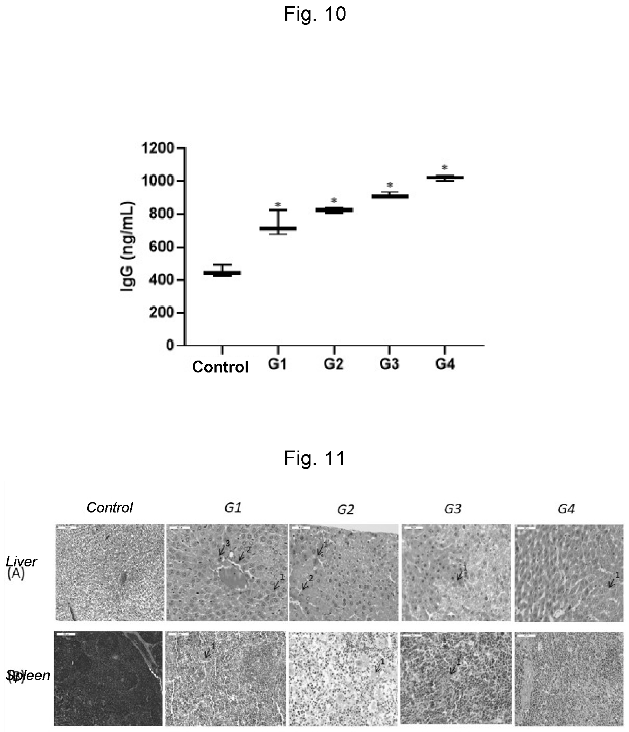

Figure 10: Figure 10 refers to *Enzyme Linked Immuno Sorbent Assay* (ELISA). Stimulation of the humoral vaccine response, data refers to the levels of total IgG produced prior to the application of the lethal dose of active alpha toxin (157 DL50.g⁻¹) in non-immunized Swiss mice (Control), of the adjuvant group (G1) and immunized groups (G2, G3 and G4). (*) *p* value p < 0.05 represents comparative analysis between vaccine groups with the control group.
Figure 11: Figure 11 refers to histopathological data from experimental groups after immunization and challenge with a 157 DL50.g⁻¹ dose stained using the hematoxylin-eosin technique. G1 (animals inoculated with adjuvant in W/O microemulsion). G2 and G3 - immunized animals ALFA-T / REC DE3 / Ep1 and ALFA-T / REC DE3 / Ep2 in W/O microemulsion, respectively. G4 - immunized animals ALFA-T / REC DE3 / Ep1 + ALFA-T / REC DE3 / Ep2 associated in W/O microemulsion (A) Data related to the organs: liver. (B) Data related to the organs: spleen. Data: A1 - Degeneration (40x), A2 - Hemorrhage (40x), A3 - Hyperemia (40x), B1 - Degeneration (40x).

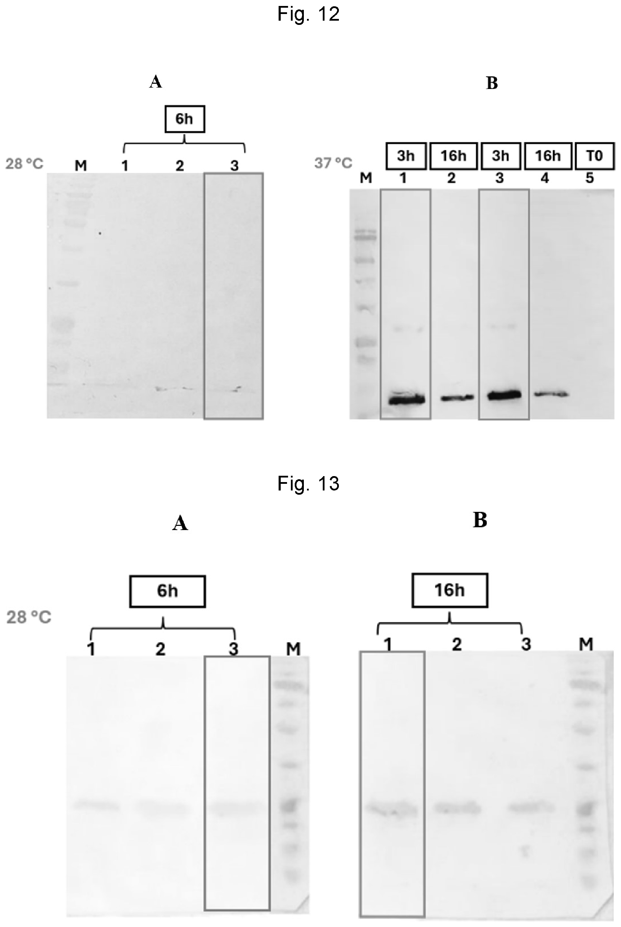

Figure 12: Figure 12 refers to the comparison of the conditions for inducing Ep1 protein. (A) Expression of the EP1 protein according to the initially proposed induction strategy, that is, in the *E. coli* Bl21 (DE3) pLysS cell, 28 °C, 6h induction, channel 1: lactose inductor, 10 g / L; channel 2: IPTG inductor, 0.2 mM; channel 3: lactose inductors (10 g / L) + IPTG 0.2 mM, the initially proposed induction strategy is highlighted with a rectangular outline. (B) Expression of the EP1 protein according to an enhanced induction strategy, that is, in the *E. coli* Tuner DE3 cell, 37 °C, channel 1: IPTG inductor, 1 mM, 3h induction; channel 2: IPTG inductor, 1 mM, 16h induction; channel 3: lactose inductor, 10 g / L, 3h induction; channel 4: lactose inductor, 10 g / L, 16h induction; channel 5: time zero, before induction. In channels 1 and 3, the expression condition after enhancement is highlighted with a rectangular outline.
Figure 13: Figure 13 refers to the comparison of the conditions for inducing protein Ep2. (A) EP2 protein expression according to the initially proposed induction strategy, that is, in the *E. coli* Bl21 (DE3) pLysS cell, 28 °C, 6h induction, channel 1: lactose inductor, 10 g / L; channel 2: IPTG inductor, 0.2 mM; channel 3: lactose inductors (10 g / L) + IPTG 0.2 Mm. In channel 3, the induction strategy described in the initially proposed patent is highlighted with a rectangular outline. (B) Expression of the EP2 protein according to an enhanced induction strategy, that is, in the *E. coli* Bl21 (DE3)pLysS cell, 37 °C, channel 1: IPTG inductor, 1 mM, 16h induction; channel 2: IPTG inductor, 0.1 mM, 16h induction; channel 3: lactose inductor, 16h induction. In channel 1, the expression condition after enhancement is highlighted with a rectangular outline.

### DETAILED DESCRIPTION OF THE INVENTION

Unless defined otherwise, all technical and scientific terms herein used have the same meaning as understood by a person skilled in the art to which the invention pertains. The terminology used in the description of the invention has the objective to describe particulars embodiments only, and it is not intended to limit the scope of the teachings. Unless indicated otherwise, all numbers expressing quantities, percentages and proportions, and other numerical values used in the specification and in the claims, should be understood as being modified in all cases, by the term "about." Thus, unless stated otherwise, the numerical parameters shown in the specification and in the claims are approximations that may vary depending on the properties to be obtained.

As used throughout the present application, the term "amino acid" means the α-amino acids group which can be encoded by a nucleic acid directly or in the form of a precursor. The individual amino acids are encoded by nucleic acids consisting of three nucleotides, known as codon or base triplets. Each amino acid is coded by at least one codon. The fact that the same amino acid is coded by different codons is known as "degeneration of the genetic code." The term "amino acid," as used in the present application, means the naturally occurring α-amino acids, comprising alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine.

The terms "peptide," "polypeptide" or "protein" can be used interchangeably and refer to an amino acids' polymer binds by peptide bonds, regardless of the number of amino acid residues that make up this chain. The polypeptides, as herein used, include "variants" or "derivatives" thereof, which refers to a polypeptide including variations or modifications, for example, substitution, deletion, addition, or chemical modifications in its amino acid sequence in relation to the reference polypeptide. Examples of chemical modifications are glycosylation, G-alkylation, PEG alkylation, phosphorylation, acetylation, amidation, etc. The polypeptide can be artificially produced from nucleotide sequences cloned using recombinant DNA technology or it can be prepared through a known chemical synthesis reaction.

More specifically, the term polypeptide of the present invention can also be understood as antigen, poly antigen or multiepitope antigen, which consist of a junction of different epitopes that may or may not be linked by flexible or rigid linkers, specific to a single pathogen or to different pathogens.

The term "epitope" refers to an antigenic determinant in a molecule, for example, a part in a molecule that is recognized by the immune system, for example, which is recognized by an antibody. For example, the epitopes are the discrete three-dimensional sites on an antigen that are recognized by the immune system. Epitopes generally consist of chemically active surface clusters of molecules, such as amino acids or sugar side chains, and usually have specific three-dimensional structural features as well as specific charge characteristics. Conformational and non-conformational epitopes are differentiated by the fact that binding to the former, but not the latter, is lost in the presence of denaturing solvents. An epitope of a protein preferably comprises a continuous or discontinuous portion of said protein and is preferably between 5 and 100, preferably between 5 and 50, more preferably between 8 and 30, more preferably between 10 and 25 amino acids in length, for example, the epitope can preferably have 8, 9, 10, 1 1, 12, 13, 14, 15, 16, 17, 18, 19, 20, 21, 22, 23, 24 or 25 amino acids in length.

In a first embodiment, the invention provides *alpha toxin from C. novyi* vaccine peptides.

The gene regions of *alpha toxin from C. novyi* were predicted and selected using the IEDB-AR platform, from GenBank database: CAA88565.1. The regions which are correspondent to the vaccine epitope DE3 / Ep1 are comprised in the N-terminal domain, with part of the hydrophobic domain in the central region for translocation of the toxin to the cytosol. The regions corresponding to the DE3 / Ep2 epitope are located within the C-terminal domain, also including part of the hydrophobic domain.

The epitopes were selected considering aspects of antigenicity, flexibility, and stimulation of the immune response in the *in-silico* model, expressed in attenuated *E. coli* revealing bands of 17 kDa and 26 kDa corresponding to DE3 / Ep1 and DE3 / Ep2, respectively (Figure 1). It should also be clarified that, after alignments are made using the *Blast* tool (https://blast.ncbi.nlm.nih.gov/Blast.cgi), no sequences identical to those of the present invention were found in the prior art.

For the expression in *E. coli*, the epitopes genes were inserted in expression plasmids. In a preferred embodiment, the vector used is selected from the group pRSET, pColdl pASK-IBA, pT7-7, pBAD-TOPO, pSC101, pACYC, and any other vector for expression in microbial cells (see figure 2).

Preferably, the vector used is pRSET.

The term "expression vector" or, simply, "vector" can be understood as DNA molecules that replicate autonomously and can be used to transport exogenous DNA fragments. It is a vehicle used in gene cloning. The DNA of interest is first cloned into an appropriate vector and then, through transfection, the gene can be inserted into the host for expression.

The expression vectors are applicable for use in microbial host organisms as disclosed in this application and include, for example, plasmids, phage vectors, viral vectors, episomes and artificial chromosomes, including vectors and selection sequences or operable markers for stable integration into a host chromosome.

Furthermore, the expression vectors may include one or more selectable marker genes and appropriate expression control sequences. Also, selectable marker genes can be included that, for example, provide resistance to antibiotics or toxins, compensate for auxotrophic deficiencies, or supply critical nutrients that are not present in the culture medium.

Expression control sequences may include constitutive and inducible promoters, transcription enhancers, transcription terminators, and similar sequences that are well known in the art. When two or more exogenous coding nucleic acids are to be co-expressed, both nucleic acids may be inserted, for example, in a sole expression vector or in separate expression vectors.

For single-vector expression, the coding nucleic acids can be operationally linked to a common expression control sequence or linked to different expression control sequences, such as an inducible promoter and a constitutive promoter.

The transformation of exogenous nucleic acid sequences involved in a metabolic or synthetic pathway can be confirmed using methods well-known in the art. Such methods include, for example, nucleic acid analysis, such as *Northern blot* or mRNA amplification by polymerase chain reaction (PCR), or immunoblotting for gene product expression, or other analytical methods suitable for assessing the expression of an introduced nucleic acid sequence or its corresponding gene product.

It is understood by those skilled in the art that exogenous nucleic acid is expressed in sufficient quantity to produce the desired product, and it is further understood that expression levels can be optimized to achieve sufficient expression using methods well known in the art and as disclosed herein.

In one example, the *E. coli* strain may be selected from the group comprising, but not limited to: C41 (DE3), Rosetta (DE3), Origami (DE3), or Turner (DE3). Preferably, the *E. coli* strain used in the present invention is BL21 (DE3) pLysE.

**Table 1:**

| Nomenclature | Peptide sequence |
|---|---|
| Ep1 (SEQ ID NO: 2) | IKNTFDMLSSQNQEIFEASNNLYFSKTHDEFKSSWLLRSNIAE KEFQKLIKTYIGRTLNYEDGLNFNKWKRVTTSELLKVIEEVNS TKIYENY |
| Ep2 (SEQ ID NO: 4) | VSGVDENNRYLEYMLSTNNKWIINGGYWENDFNNYKIVDFEKC NVIVSGSNKLNSEGDLADTIDVLDKDLENLYIDSVIIIPKVYT KKIIIHPIPNNPQINIINTQSIHDKCHLIIDSVLTNNYHWESD GDDLIITNGLDINIRILQGLSFGFKYKNIYLKFSNYDELSLND FLLQNYNVK |

**Table 2:**

| Nomenclature | Nucleotide sequence |
|---|---|
| Ep1 (SEQ ID | ATCAAAAACACCTTTGATATGCTGAGCAGCCAGAATCAAGAAA |
| NO: 1) | TTTTTGAAGCCAGCAACAACCTGTACTTTAGCAAAACCCATGA TGAGTTTAAAAGCAGCTGGCTGCTGCGTAGTAATATTGCCGAA AAAGAATTTCAGAAACTGATCAAAACCTACATTGGTCGCACCC TGAATTATGAAGATGGCCTGAATTTCAACAAATGGAAACGTGT TACCACCTCCGAACTGCTGAAAGTTATTGAAGAAGTTAACAGC ACCAAAATCTATGAAAATTAT |
| Ep2 (SEQ ID NO: 3) | GTTAGCGGTGTTGATGAAAATAATCGCTATCTGGAATATATGC TGAGCACCAACAACAAGTGGATTATTAACGGTGGCTATTGGGA GAACGATTTCAACAACTATAAAATCGTGGACTTTGAAAAATGC AACGTGATTGTTAGCGGCAGCAATAAACTGAATAGCGAAGGTG ATCTGGCAGATACCATTGATGTTCTGGATAAAGATCTGGAAAA CCTGTATATCGATAGCGTGATTATTATCCCGAAAGTGTACACC AAGAAAATCATCATTCATCCGATTCCGAATAACCCGCAGATTA ACATTATTAACACCCAGAGCATCCACGATAAATGCCATCTGAT TATTGATAGCGTGCTGACCAATAACTATCATTGGGAAAGTGAT GGTGATGACCTGATTATTACCAATGGCCTGGATATTAACATTC GCATTCTGCAGGGTCTGAGCTTTGGCTTTAAATACAAAAACAT CTATCTGAAATTCAGCAACTACGATGAACTGAGCCTGAACGAT TTTCTGCTGCAGAATTATAACGTGAAA |

Reactivity with serum from mice infected with wildtype *alpha toxin from C. novyi* revealed the ability of these epitopes to recognize specific antibodies present in the infection (Figure 1).

The determination of the DE3 inoculum fraction was conducted in orbital shakers and SD (semi-defined) medium at proportions of 2.5, 5, and 10 % (v/v). Response surface modeling data revealed that the 10 % (v/v) proportion allowed for greater biomass yield and specific growth rate, conditions adopted in subsequent studies (Figure 3).

Aiming to optimize the production of DE3 / Ep1 and DE3 / Ep2 epitopes, we evaluated DE3 cultures in a 1L mechanically stirred tank biological reactor, which can be conducted in batch, fed batch, or continuous mode, as described in this document.

The kinetics were conducted with a working volume of 700 mL, with three (3) lactose induction pulses of 10 g / L, with the induction time maintained for 6 h after reaching maximum cell density (Figure 4).

The kinetic profile of DE3 in a single batch revealed DCW (Dry Cell Weight) yield of 0.70 and 0.77 g / L, with adequate O2 supply (%) and total glucose depletion, no significant production of Hac (g / L) (acetic acid) was evidenced (Fig. 3AC). Specific growth rate (µ), yield coefficient (Y_{X/S} and Y_{P/S}) and volumetric productivity of products and cells (Q_{P} and Q_{X}), do not differ from each other regarding DE3 cultures aimed at producing the DE3 / Ep1 and DE3 / Ep2 epitopes (Table 3).

**Table 3:**

| Gene name | Sequenc e type | Cloning vector expresses: | Syst em | C e l l | Bior eact or | Indu ctio n | Performance coefficient | | | | | |
|---|---|---|---|---|---|---|---|---|---|---|---|---|
| | | | | | | | *µ ( h^{- 1})* | *P (mmo l*/*mL* | *Y x*/*s (g*/*m mol• mL)* | *Y* _{*P*/}*_{S} (mmol*/*mL• (mmol*/*mL) ⁻¹)* | *Q_{P} (mmo l*/*mL •h)* | *Q_{X} (g*/*mL •h)* |
| Alpha toxin (DE3 / Ep1) | Protein | pRSET A | Batc h | D E 3 | Lab scal e | lact ose | 0 . 2 4 | 0.56 | 30.4 6 | 0.26 | 0.03 | 8.49 |
| | | | Fed-batc h | D E 3 | Lab scal e | lact ose | 0 . 2 8 | 0.56 | 62.0 7 | 0.35 | 0.03 | 12.49 |
| Alpha toxin (DE3 / Ep2) | Protein | pRSET A | Batc h | D E 3 | Lab scal e | lact ose | 0 . 2 5 | 0.61 | 34.3 5 | 0.29 | 0.03 | 9.15 |
| | | | Fed-Batc h | D E 3 | Lab scal e | lact ose | 0 . 2 9 | 0.61 | 61.2 8 | 0.37 | 0.04 | 12.73 |

The kinetic profile of DE3 in fed-batch, under these same induction conditions, revealed a yield of DCW of 1.03 g / L, with adequate O2 supply (%) and total glucose depletion, also with no evidence of significant HAC production (g / L) (Fig. 3BD). Specific growth rate (µ), performance coefficient (Y_{X/S} and Y_{P/S}) and volumetric productivity of products and cells (Q_{P} and Q_{X}), do not differ from each other regarding DE3 cultivation aimed at producing epitopes DE3 / Ep1 and DE3 / Ep2, however, all were superior to those obtained in the simple batch (Table 3).

The scaling up of epitope vaccine production was conducted in a fed-batch system, as this represented the highest yields obtained in previous studies. Therefore, we established the scaling up of epitope production by determining the yield in a 20 L non-stirred tank biological reactor, with the expectation of reducing operational costs.

The kinetics were conducted with a working volume of 10 L, with three (3) induction pulses associated with a low concentration of isopropyl-β-D-thiogalacto pyranoside (IPTG) inducer (0.2 mM) with 10 g / L lactose solution, the induction time being maintained for 6 hours after reaching maximum cell density (Figure 5). The kinetic profile of DE3 in fed-batch revealed similar parameters between the productions of vaccine epitopes (DE3 / Ep1 and DE3 / Ep2), with an DCW yield of 1.07 and 1.20 g / L being achieved. The values of O2 (%), glucose consumption (g / L), and Hac production (g / L) showed profiles similar to those described in previous studies (Figure 5AB).

Specific growth rate (µ), performance coefficient (Y_{X/S} and Y_{P/S}) and volumetric productivity of products and cells (Q_{P} and Q_{X}) do not differ from each other regarding DE3 culture aimed at producing the epitopes DE3 / Ep1 and DE3 / Ep2, however, all were superior to those obtained in the mechanically stirred fed-batch process (Table 4).

The induction strategy using 10 g / L lactose pulses revealed production profiles of DE3 / Ep1 and DE3 / Ep2 in both processes (simple batch and fed batch) conducted in a mechanically stirred tank biological reactor with a volume of 1 L. Peak production of DE3 / Ep1 and DE3 / Ep2 is revealed in the ranges of 300 mAU (simple batch) to 400 mAU (fed batch), indicating higher production in the fed batch system (Figure 6).

In the 10 L scaling, we used a combination of 10 g / L lactose and 0.2 mM IPTG, which revealed increments. Peaks production of DE3 / Ep1 and DE3 / Ep2 were revealed in the 1000 mAU range (Figure 7A) and 600 mAU (Figure 7B), respectively.

The lactose and IPTG combination strategy has shown promise and can be explored by evaluating different combinations in future scaling-up studies for recombinant protein induction in DE3 cultures. The purified epitopes of 17 kDa (DE3 / Ep1) and 26 kDa (DE3 / Ep2) were obtained with a concentration corresponding to 100 ng / µl and 110 ng / µl, respectively (Figure 1, Table 4).

**Table 4:**

| Gene name | Seque nce type | Cloni ng vecto r expre sses: | System | Cel l | Biore actor | Indu ctio n | Performance coefficient | | | | | |
|---|---|---|---|---|---|---|---|---|---|---|---|---|
| | | | | | | | *µ (h ⁻¹)* | *P (mmol*/ *mL)* | *Y*_{/}*_{S} (g*/*mmol •mL)* | *Y*_{*P*/}*_{S} (mmol*/*m L•(mmol* /*mL)⁻¹)* | *Q_{P} (mmol*/*m L•h)* | *Q_{X} (g*/*m L•h)* |
| Alpha toxin (DE3 / Ep1) | Prote in | pRSET A | Fed-batch | DE3 | Scale up | Lact ose / IPTG | 0. 30 | 0.56 | 65.76 | 0.36 | 0.04 | 12.9 7 |
| Alpha toxin (DE3 / Ep2) | Prote in | pRSET A | Fed-batch | DE3 | Scale up | Lact ose / IPTG | 0. 31 | 0.61 | 71.85 | 0.38 | 0.04 | 14.5 7 |

Additionally, seeking to improve the expression of recombinant proteins, new induction strategies for obtaining Ep1 and Ep2 proteins were evaluated. Different temperatures (11, 22, and 37 °C), times (3h and 16h), competent cells (*E. coli* Bl21 (DE3) pLysS, *E. coli* Tuner DE3, *E. coli* Arctic Xpress DE3) and inductors (1 mM IPTG, 0.1 mM IPTG and 10 g / L lactose) were assessed.

Specifically, it was observed that when the conditions of 37 °C, *E. coli* Tuner DE3, 1 mM IPTG, or 10 g / L lactose were applied for 3 hours, the Ep1 protein showed improved induction compared to the initially proposed induction strategy, as can be seen in Figure 12B, channels 1 and 3.

It is important to emphasize that the lactose inducer is a non-toxic and low-cost reagent when compared to IPTG, thus being ideal for expression in large volumes. The approximate yield obtained in the improved induction strategy was 10 mg / L, while in the initially proposed strategy it was approximately 0.05 mg / L.

Regarding the Ep2 protein, an improvement in induction was also observed, compared to the initially proposed strategy, when the conditions of 37 °C, *E. coli* Bl21 (DE3) pLysS, 1 mM IPTG, for 16 hours of induction were employed, as can be seen in Figure 13B, channel 1.

The lactose inductor (10 g / L) produced similar expression levels after the same test time (figure 13B, channel 2). It is important to emphasize that the lactose inducer is a non-toxic and low-cost reagent when compared to IPTG, therefore being ideal for expression in large volumes. The approximate yield obtained in the improved induction strategy was 5 mg / L, while in the initially proposed strategy it was approximately 0.05 mg / L.

On the other hand, expressions using the *E. coli* Arctic Xpress DE3 strain did not show satisfactory results under any of the conditions assessed.

Thus, the improved induction strategies assessed proved promising when employed in *E. coli* Tuner DE3 and *E. coli Bl21* (DE3) pLysS.

In a second embodiment, the invention provides a vaccine composition comprising the alpha toxin epitopes of *C. novyi*.

In a preferred embodiment, the vaccine composition comprises the DE3 / Ep1 and DE3 / Ep2 epitopes as revealed herein, preferably in the concentrations described in this application as an antigenic component, which is mixed with an adjuvant.

Adjuvants are also useful for improving the immune response and / or increasing the stability of the vaccine preparations. Adjuvants are typically described as non-specific stimulators of the immune system, but they can also be useful for targeting specific arms of the immune system. One or more compounds possessing this activity may be added to the vaccine.

Thus, specific vaccines of the present invention further comprise an adjuvant. Examples of chemical compounds that can be used as adjuvants include, but are not limited to, aluminum compounds (for example, aluminum hydroxide), metabolizable and non-metabolizable oils, mineral oils, including mannet oleate derivatives in mineral oil solution (for example, MONTANIDE ISA 61), and light mineral oils, such as DRAKEOL 6VR, block polymers, ISCOM (immunostimulant complexes), vitamins and minerals (including, among others: vitamin E, vitamin A, selenium and vitamin B12) and CARBOPOL^{®}.

Preferably, the adjuvant is MONTANIDE^{™} ISA 61 VG, Seppic, Brazil.

Besides, the vaccine compositions of the present invention may include diluents, isotonic agents, stabilizers, or adjuvants. Diluents may include water, saline solution, dextrose, ethanol, glycerol, and the like. Isotonic agents may include sodium chloride, dextrose, mannitol, sorbitol, and lactose, among others. Stabilizers include albumin and alkaline salts of ethylenediamine tetra acetic acid, among others. Suitable adjuvants are known in the art.

Furthermore, according to the invention a "immune or immunologic response" to a composition or vaccine is the development in the host of a cellular and / or antibody-mediated immune response to the composition or vaccine of interest. Typically, an "immune response" includes, but is not limited to, one or more of the following effects: the production or activation of antibodies, B cells, helper T cells, suppressor T cells and / or cytotoxic T cells and / or gamma-delta T cells, specifically directed against an antigen or antigens included in the composition or vaccine of interest. Preferably, the host will exhibit a therapeutic or protective immune response, such that resistance to new infection is increased and / or the clinical severity of the disease is reduced. This protection will be demonstrated by a reduction or absence of symptoms associated with host infections.

In a third embodiment, the invention provides the use of such vaccine epitopes in the preparation of a vaccine composition for the prevention and treatment of the damage provoked by the active *Alpha toxin from C. novyi* type B.

The predicted and selected protein regions of the *alpha toxin from C. novyi* revealed potential for epitope selection, with the region corresponding to DE3 / Ep1 located on N-terminal domain and a region DE3 / Ep2 belonging to the C-terminal domain. Both in the *in-silico* model and after expression in DE3, the epitopes DE3 / Ep1 and DE3 / Ep2 revealed bands of 17 kDa and 26 kDa respectively (Figure 1D).

Furthermore, the reactivity of these was evidenced with serum from mice infected with wildtype *alpha toxin from C. novyi*, which highlights the importance of these selected regions in recognizing specific antibodies present in the infection (Figure 1E).

Post-immunization follow-up revealed that the entire experimental group experienced weight gain, demonstrating aspects of the formulation's safety (Figure 8A). Post-challenge hematological data were used to assess changes in hematological parameters and liver function (Table 5).

Table 5 presents hematological data from experimental groups after immunization and challenge with 157 DL50.g⁻¹ dose. G1 (animals inoculated with adjuvant in W/O microemulsion). G2 and G3 - immunized animals ALFA-T / REC DE3 / Ep1 and ALFA-T / REC DE3 / Ep2 in W/O microemulsion, respectively. G4 - immunized animals ALFA-T / REC DE3 / Ep1+ALFA-T / REC DE3 / Ep2 COMBINED in W/O microemulsion. Control (-) non-immunized animals / not challenged (healthy animals); Control (+) non-immunized animals challenged with 157 DL50.g⁻¹ doses. Ns - not significant - sera from non-immunized / non-challenged mice vs. sera from mice inoculated with adjuvant in microemulsion (Group G1). *Significant - hemoglobin (p < 0.05); hematocrit (p < 0.05) sera from immunized mice (G2, G3 and G4) vs positive control.

**Table 5:**

| Parameters | G1 group | G2 group | G3 group | G4 group | Control (+) | Control (-) | Reference |
|---|---|---|---|---|---|---|---|
| Leukocytes | 9600 (864)^{ns} | 7002 (809) | 7066 (907) | 7133 (503) | 12933 (7128) | 8466 (982) | 4300 - 10000 |
| Hemoglobin (g/dL) | 14.73 (0.51)^{ns} | 12.23 (0.5) | 12.67 (0.6) | 12.00 (1.0) | 20.73 (1.80) * | 10.60 (2.80) | 13 - 17 |
| Hematocrit (%) | 44.33 (1.53)^{ns} | 39.13 (1.3) | 39.33 (1.5) | 39.17 (1.0) | 64.67 (3.05) * | 38.80 (4.50) | 39 - 51 |

The positive control group presented clinical signs of polycythemia; in contrast, immunized mice from groups (G2), (G3), and (G4) showed data in accordance with reference values and comparatively similar to data from healthy animals (Table 5).

Vaccine efficacy data revealed that animals in the control and adjuvant (G1) groups did not differ from each other, although a high mortality rate was observed after the challenge test (Figure 9).

The animals immunized with microemulsion using DE3 / Ep1 (G2) and DE3 / Ep2 (G3) revealed a protection rate of 25 % and 40 %, respectively. A microemulsion composed of combined epitopes DE3 / Ep1 and DE3 / Ep2 (G4) allowed for a higher protection rate, at 75 %.

Vaccines from groups G3 and G4 provided superior efficacy data compared to those obtained using microemulsions containing inactivated wildtype alpha toxin epitope (G5), with 25 % (Félix et al. 2019) (P < 0.005), whereas the microemulsion containing DE3 / Ep1 epitope (G2) provided the same protection to animals as G5.

Our data showed that the obtained vaccine epitopes can be used as immunogens in vaccine protection against damage caused by the active *alpha toxin from C. novyi* type B.

However, the hematological findings (Table 5) are confirmed by hematological data (Figure 8) and also correlate with vaccine efficacy data (Figure 9), as well as in ELISA analysis data from liver and spleen of mice challenged with active *alpha toxin from C. novyi* type B (Figure 10).

This evidence suggests that *in silico* analysis of antigenic regions, followed by *in vivo* experiments, may be an effective strategy for considering a suitable candidate for the development of vaccines against *C. novyi* type B. However, advances in epitope yield optimization studies are needed to enable adjustments in the concentration of each antigen in the vaccine dose. Histopathological analysis of liver and spleen tissue damage corroborated our findings, indicating the safety of the vaccine groups, with group G4 showing greater effectiveness. Similarly, the ELISA immunogenicity test, used to verify antibody production, indicated a greater capacity to stimulate an immune response for vaccine group G4.

### Examples

### Example 1 - Bioinformatics tools and construction of expression vector of vaccine epitopes

Computer programs were used for selection of epitopes of *alpha toxin from C. novyi* (*alpha toxin from Clostridium novyi* - GenBank: CAA88565.1). The regions were predicted using *Immune Epitope Database Analysis Resource* (IEDB-AR) (http://tools.immuneepitope.org/main), selecting two alpha toxin epitopes located on amino acid 491° to 583° of the N-terminal region (DE3 / Ep1), and amino acids 1599° to 1779° of the C-terminal region (DE3 / Ep2), both obtained by considering the relationship between antigenicity, flexibility, and the ability to stimulate an adaptive immune response.

The DE3 / Ep1 and DE3 / Ep2 genes were synthesized by Invitrogen - Thermo Fisher Scientific^{®}, with codon bias for *E. coli* inserted into the pRSET expression plasmid, which allows the addition of a poly histidine tail (6xHIS) at the amino-terminal end of each protein, in order to facilitate purification after cell lysis.

The plasmid containing the synthetic gene was transformed into electrocompetent DE3 using the 2510 electroporator (Eppendorf^{®}), following the manufacturer's instructions.

Next, the cells were plated on Luria Bertani (LB) culture medium (Kasvi^{®}) containing ampicillin (100 µg / mL) (Multilab^{®}) and chloramphenicol (11.4 µg / mL) (Kasvi^{®}) and incubated overnight at 37 °C. Epitope expression in DE3 was performed using isopropyl-β-D-thiogalactopyranoside (IPTG) induction (Invitrogen^{®}) in LB medium with ampicillin and chloramphenicol.

The cultures were conducted in the early hours until growth of DO600 (0.6) at 37 °C, and after, induction with 1 mM IPTG was performed, followed by temperature adjustment to 28 °C. Samples of DE3 cultures were treated with denaturing buffer (20 % (v / v) glycerol (Synth^{®}); 4 % (w / v) sodium dodecyl sulfate (SDS) (Synth^{®}); 100 mM Tris pH 6.8 (Synth^{®}); 0.2 % (w / v) bromophenol blue (Synth^{®}); 200 mM beta-mercaptoethanol (BioRad^{®})), and subsequent boiling treatment for 10 min.

Polyacrylamide gel electrophoresis (SDS-PAGE 12 %) was performed to confirm the expression of the corresponding epitopes of the DE3 / Ep1 and DE3 / Ep2 genes, followed by *western blotting*.

The design and expression of the DE3 / Ep1 and DE3 / Ep2 in DE3 were conducted in partnership with the Laboratory for Diagnosis and Control of Infectious Diseases of the Amazon (LDCDIA), of the Leônidas & Maria Deane Institute (ILMD / Fiocruz Amazônia).

### Example 2 -DE3 culture in a rotary shaker

Isolated colonies of DE3 were inoculated into semi-defined (SD) culture medium supplemented with 100 µg / mL of ampicillin. The SD medium consisted of glucose 5.0 g / L (Synth^{®}); yeast extract 5.0 g / L (Synth^{®}); dibasic potassium phosphate (K₂HPO₄) 9.4 g / L (Synth^{®}); monobasic potassium phosphate (KH₂PO₄) 5.0 g / L; sodium chloride (NaCl) 5.0 g / L (Synth^{®}), zinc chelate (EDTA-Zn) 1.0 g / L (Comnagro^{®}) and manganese chelate (EDTA-Mn) 0.2 g / L (Comnagro^{®}).

The studies were conducted in triplicate using a rotary shaker SL-223/F (Solab Cientifica^{®}) controlled at 37 °C, 200 rpm for 16 h. Inoculum fractions corresponding to 2.5 %, 5 %, and 10 % were evaluated by collecting samples, taking optical density readings (OD600), and determining the specific growth rate (µh⁻¹) and DCW.

### Example 3 -DE3 culture in SD medium in a stirred tank biological reactor.

Batch and fed-batch cultures were conducted in a Tecnal BIO-TEC^{®} 1 L stirred tank biological reactor, consisting of a jacketed glass vessel; a digital control unit; and pH control at 7 using 3 M sodium hydroxide (NaOH) (Synth^{®}) at 37 °C; and a dissolved oxygen (DO) supplementation strategy, with cascade agitation control from 100 - 1000 rpm, and a fixed specific air feed flow rate of 3 vvm.

Inoculum propagation was initially performed using a stock culture of each DE3 (1.5 mL) activated in 8.5 mL of SD medium, under agitation at 200 rpm for 12 h at 37 °C, maintaining a 10 % inoculum fraction in the bioreactor.

During the fed-batch process, 7 mL of 500 g / L glucose solution containing 100 µg / mL ampicillin were added at every 30-minute intervals until reaching a stationary growth phase. Samples were then collected at 1-hour intervals to determine growth kinetics, acetate and glucose consumption, as well as synthesis of DE3 / Ep1 and DE3 / Ep2.

### Example 4 - Scaling up of DE3 culture in SD medium in a non-stirred tank biological reactor

Fed-batch cultures were conducted in a 20 L non-stirred tank biological reactor, consisting of a wide-mouthed round bottle (Thermo Scientific Nalgene^{®}) adapted from a round carboy, with low-density polyethylene (LDPE) handles to facilitate transport and dispensing, and a white polypropylene (PP) screw cap (83B).

The cultures were conducted with a pH control of 7 using 3 M NaOH at 37 °C; and a dissolved oxygen (DO) supplementation strategy with a fixed air intake of 3 vvm, without mechanical agitation. Inoculum propagation was performed using a stock culture of each DE3 (1.5 mL) activated in 8.5 mL of SD medium, under agitation at 200 rpm for 12 h at 37 °C, maintaining a 10 % inoculum fraction for cultivation in the bioreactor with a working volume of 10 L.

During the fed-batch process, 100 mL of a 500 g / L glucose solution supplemented with 100 µg / mL ampicillin was added at every 30-minute intervals until reaching a stationary growth phase. Samples were then collected at 1-hour intervals to determine growth kinetics, acetate and glucose consumption, as well as for synthesis of DE3 / Ep1 and DE3 / Ep2.

### Example 5 - Induction and purification system for recombinant DE3 / Ep1 and DE3 / Ep2 epitopes.

In the first induction stage, we used 3 pulses of 35 mL of 10 g/L lactose solution (Synth^{®}) in the cultures conducted in the stirred tank biological reactor, immediately after reaching maximum cell growth, followed by another addition two hours later. In the scale-up using a non-stirred tank biological reactor, we used 3 pulses of 500 mL of the 10 g / L lactose solution with 0.2 mM IPTG, at the same time intervals applied in the lab-scale experiments.

All induction studies were conducted for 6 h at 28 °C. A chromatography system (Akta Purifier 10^{®}, GE Lifescience Healthcare^{®}) was used in the step of purification of DE3 / Ep1 and DE3 / Ep2. The collected fractions were lyophilized and stored at -20 °C for analysis. The reversed-phase chromatography was performed according to what was proposed by Field et al. (2021).

DE3 / Ep1 and DE3 / Ep2 fractions, obtained from affinity chromatography, after lyophilization, were solubilized in 0.1 % trifluoroacetic acid (TFA) (solution A) (Merck^{®}) and subjected to high-performance liquid chromatography (HPLC) on a C-18 column (25 mm x 4.6 mm, Supelco^{®}), previously equilibrated with solution A and eluted under a gradient from 0 to 70 % with solution B (acetonitrile 99.9 % (Merck^{®}) and TFA 0.1 %) in 5 column volumes, under flow of 1 mL / min.

The elutions were monitored at 280 nm. The profiles referring to DE3 / Ep1 and DE3 / Ep2 were obtained confirming the degree of purification achieved and the concentration of the product. The collected fractions were again visualized on 12 % SDS-PAGE to check purity and confirm antigenicity by western blotting.

### Example 6 - Western blotting

Bands referring to DE3 / Ep1 and DE3 / Ep2, present in 12 % SDS-PAGE gel, were transferred to nitrocellulose membranes following the BioRad^{®} protocol. A primary antibody derived from the blood serum of Swiss mice infected with wildtype *alpha toxin from C. novyi* type B was used. In the negative control, blood serum from healthy Swiss mice was used.

The secondary antibody anti-mouse IgG-rabbit A9044 (Sigma^{®}) (conjugated with peroxidase in a 1:2,000 ratio and 0.05 % (v / v) of the substrate 3,3'-diaminobenzidine (DAB) was used to certify its reactivity with purified DE3 / Ep1 and DE3 / Ep2, compared to inactive wildtype alpha toxin.

### Example 7 - Fermentative parameters

**Example 7.1 - Determination of glucose consumption**

The determination of glucose consumption was conducted using the liquid enzymatic glucose system (Labtest^{®}) Eq. (1), where the principle consists of two reactions using the enzymes glucose oxidase (GOD) and peroxidase (POD) as described by Bergmeyer et al. (1986).

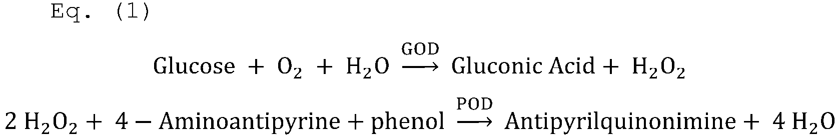

The product formed, 4-antipyrliquinonimine, has a reddish color, its intensity being directly proportional to the glucose concentration. The assays were prepared in 2 mL shaken tubes, incubated for 5 min at 37 °C, and the reading was taken at 510 nm. The glucose concentration was calculated as described in: $Glucose = \frac{Abs test}{Abs standard} ⋅ 100 \left(mg/dL\right)$

### Example 7.2 - Determination of Hac and DCW production

The determination of Hac was performed using a Shimadzu^{®} chromatograph (SPD - M2OA), equipped with a high-precision LC-6AD pump (CBM-20ª) and ultraviolet detectors that detect in the absorbance range of 190 to 700 nm for Hac detection. The ion-exclusion column used was the Shim-pack^{®} C-18 column (250 x 4.6 mm, Shimadzu^{®}). The analysis was conducted for 12 min at 30 °C with a mobile phase flow rate of 0.6 mL / min. The mobile phase consisted of a 1 % phosphoric acid (H₃PO₄) solution with an injection volume of 20 µL. Samples were filtered using a Chromabono^{®} C-18 membrane (3 mL / 500 mg), and readings were taken at 204 nm. Hac determination was performed using a standard curve and linear regression.

DCW determination was obtained by the difference between mass A (mA) and mass B (mB), where mA represents the mass of the crucibles without sample, mB represents the mass of the crucibles containing culture samples, and Al represents the sample volume. DCW values were generated by correlating the dry weights and OD600 of culture samples, where one (1) OD600 unit corresponds to 0.61 g / L of dry weight of the cells. The dry mass correlation was determined as described in Eq. (3). $DCW = \frac{\left(mA-mB\right)}{Al} ⋅ 1000 \left(g/L\right)$

### Example 7.3 - Performance coefficients

The specific growth rate (µ) was determined during the exponential phase of bacterial growth, in which the specific growth rate is constant and maximum (µx = µm). µ values were obtained using Eq. (4), where dX corresponds to the difference in OD600 at a specific time point and a subsequent time point, dt corresponds to the time variation, and X corresponds to the cell concentration at that specific time point. $μ = \left(\frac{dX}{dt}\right) / X \left({h}^{- 1}\right)$

Performance coefficients (*y*) were analyzed for biomass ( ${Y}_{\frac{X}{S}}$) and product ( ${Y}_{\frac{P}{S}}$)*,* viewed in Eqs. (5) and (6). Cell growth was considered in calculating the biomass-to-product conversion factor (*X*) and the substrate consumption (*S*), which were related by Eq. (5). For the calculation of product yield, the amount of product formed (*P*) and the substrate consumption (*S*) were considered, which were related by Eq. (6). Eq. (7) and (8) define the volumetric productivity of the product (*Q_{P}*) and the biomass (*Q_{X}*)*,* in relation to the time (*t*)*.* ${Y}_{\frac{X}{S}} = \frac{{X}_{f} - {X}_{i}}{{S}_{i} - {S}_{f}} \left(g/mmol.mL\right)$ ${Y}_{\frac{P}{S}} = \frac{{P}_{f} - {P}_{i}}{{S}_{i} - {S}_{f}} \left(mmol/mL {\left(mmol/mL\right)}^{- 1}\right)$ ${Q}_{P} = \frac{{P}_{f} - {P}_{i}}{t} \left(mmol/mL.h\right)$ ${Q}_{X} = \frac{{X}_{f} - {X}_{i}}{t} \left(g/mL.h\right)$

### Example 8 - Vaccine Formulation

Purified DE3 / Ep1 and DE3 / Ep2 were emulsified in adjuvant MONTANIDE^{™} ISA 61 VG, Seppic, Brazil. The aqueous phase of the vaccine (W) consisted of 40 % recombinant antigen and the oil phase (O) of 60 % adjuvant. The microemulsion was performed using sterilized 10 mL syringes connected to each other by silicone tubing. Volumes of the aqueous and oil fractions were forcibly transferred between the syringes for 10 minutes.

### Example 9 - Experimental Design

Swiss mice of both sexes, aged 4-6 weeks and weighing between 17-23 g, were used after approval by the Ethics Committee on the Use of Animals (CEUA) of the Federal University of Tocantins (UFT), no. 23.101.006.832/2017-53.

The experimental groups were separated into 5 (five) groups, with 10 (ten) animals per group, the groups being: PBS (non-immunized); Adjuvant in W/O microemulsion (G1); DE3 / Ep1 vaccine in W/O microemulsion (G2); DE3 / Ep2 vaccine in W/O microemulsion (G3); DE3 / Ep1 + DE3 / Ep2 vaccine in combination and W/O microemulsion (G4). The groups were also compared with groups immunized with wildtype inactivated alpha toxin (G5) (Félix et al., 2019).

Each component was administered intramuscularly in 3 (three) doses of 100 µL, with 15-day intervals between each application, and the weight of each animal was monitored during the experimental period.

The groups were challenged with a challenge dose (157 DL50.g⁻¹ - lethal dose active alpha toxin) and the Kaplan-Meier survival curve was determined. Blood samples were collected for specific antibody, hematological, and biochemical analyses. Livers and spleens of animals were also collected for histopathological analyses.

The surviving animals were previously anesthetized and euthanized with ketamine (300 mg / kg) (Vetbrands, Brazil) and xylazine (22.5 mg / kg) (Syntec^{®}, Brazil), and consciousness was certified by heartbeat and respiratory movements.

### Example 10 - Immunogenicity, hematological, and biochemical data

Immunogenicity was determined by analyzing sera from immunized Swiss mice using ELISA immunoassay. Blood samples from mice after the last immunization (45D) were evaluated for stimulation of total IgG production. ELISA was applied using purified DE3 / Ep1 and DE2 / Ep2 proteins for standardization and antigen fixation in 96-well microplates (Nunc MaxiSorp) at 4 °C.

After blocking for 1 hour with 2 % casein-PBS buffer (blocking buffer) at room temperature, the plates were washed four times with 0.05 % Tween-20-PBS buffer. The serum was diluted at 1:50 with blocking buffer and incubated for 16 h at 4°C.

After washing, the anti-mouse IgG peroxidase conjugate (Sigma, USA) was used in a 1:2000 ratio in the blocking buffer. After washing, 3,3',5,5'-tetramethyl benzidine (BD Biosciences, USA) was added for 15 min and blocked with 2.5 M H₂SO₄ (Dinâmica, Brazil). Optical density was measured at 450 nm, and values above the cutoff point were considered positive.

The mean optical density of the serum determined the cutoff point before immunization. Sera from non-immunized animals were used to calculate the cutoff. In addition, hematological and biochemical data were obtained by centrifuging blood in microtubes at 3500 rpm for 15 minutes, evaluating parameters of the Leukogram (Total Leukocytes, Neutrophils, and Monocytes) and Erythrogram (Erythrocytes, Hemoglobin, Hematocrit, VCM, HCM, CHCM and platelets).

### Example 11.1 - Histopathology

We evaluated the total IgG levels produced by mice challenged with active alpha toxin from *C. novyi* type B using the Enzyme-Linked Immunosorbent Assay (indirect ELISA) technique, according to the protocol established by the Laboratory for Diagnosis and Control of Infectious Diseases of the Amazon (LDCDIA).

To capture IgG, we sensitize the plate with the antigen (inactivated alpha toxin from *C. novyi* type B, in a concentration of 4 µg / ml, diluted in carbonate / bicarbonate buffer pH 9.6 and left overnight at 4 °C. After incubation, the plate was blocked with 1x PBS / 3 % BSA for 2 hours, followed by washing with 0.05 % PBS Tween before adding the sera (1:1) from mice challenged with active alpha toxin. After adding the primer, the plate was incubated in a humid chamber in an incubator at 37 °C for 1 hour, followed by washing. Subsequently, a peroxidase-conjugated anti-IgG and mouse polyclonal antibody from Rhea Biotech^{®} was applied for the development step. Finally, 100 µL of the chromogenic substrate Tetramethylbenzidine (TMB - Sigma^{®}) was added to each well and allowed to react for 10 minutes, stopping the reaction with 2M sulfuric acid (H₂SO₄).

Optical density (OD) was determined using an ELISA reader (GLOMAX^{®}) with a 450 nm filter. To determine antibody levels, an antibody titration curve was performed using mouse IgG from Rhea Biotech^{®} at different concentrations (1000 ng, 500 ng, 250 ng, 125 ng, 62.5 ng and 31.25 ng).

### 11.2 Histopathological Analysis

Liver and spleen of Swiss mice from each experimental group were removed by necropsy and fixed in 10 % buffered formalin for histopathological analysis. The tissues were processed and embedded in paraffin and stained with H&E staining.

The main liver lesions, such as hydropic degeneration of hepatocytes, inflammation, perivasculitis, hyperemia, and necrosis, were evaluated. In the spleen, periarteriolar and follicular hyperplasia in white and red pulps, and inflammatory infiltrates were assessed.

Each animal was analyzed according to the degree of lesions following the scoring system: 0) no lesions; 1) mild lesions up to 25 % of the observation field; 2) moderate lesions greater than 25 % and less than 50 % of the observation field; 3) severe lesions greater than 50 % of the observation field.

Analysis of the humoral response post-immunization revealed a greater boost in IgG levels for group G4 (combined epitopes DE3 / Ep1 and DE3 / Ep2), with values close to 1000 ng / mL, significantly higher levels than the IgG levels obtained in the Control (450 ng / mL), G1 (adjuvant) (700 ng / mL), G2 (DE3 / Ep1) (820 ng / mL) and G3 (DE3 / Ep2) (915 ng / mL) groups (p < 0.05), demonstrating a greater capacity to stimulate the immune response against *C. novyi* type B (Figure 11).

The sepsis model of infection with active alpha toxin from *C. novyi* type B showed tissue damage in the livers and spleens of mice after immunization and challenged with a dose of 157 LD50.g⁻¹. Histopathological findings revealed greater severity of degeneration, hemorrhage, and hyperemia in groups G1 (adjuvant), G2 (DE3 / Ep1), and G3 (DE3 / Ep2). In contrast, discrete alterations were observed in group G4 (epitopes associated with DE3 / Ep1 and DE3 / Ep2), similar to the data observed in the Control group (Figure 11).

### Example 12 - Statistical Analysis

The experiments were conducted following a completely randomized design employing variance analysis (ANOVA). Kinetic parameters obtained in agitated and non-agitated systems were analyzed comparatively.

The PBS (non-immunized) vaccine group was used for comparison with the immunized groups. Data means were analyzed using Student's t-test, considering a p-value (P < 0.05) as significant, using Origin Pro^{®} 8.5 software. The Kaplan-Meier survival curve defined the probability of vaccine efficacy.

### Example 13 - Transformation and Expression of EP1 and EP2 proteins in E. coli BL21 (DE3) pLysS

The transformation of *Escherichia coli* BL21 (DE3) pLysS^{™} bacteria with pRSET plasmids containing the EP1 and EP2 genes was performed following this procedure:

First, 0.5 µL (corresponding to 25 ng) of the plasmid was added to a total of 50 µL of competent *E. coli* BL21 (DE3) pLysS cell. Subsequently, this mixture was exposed to an electrical pulse with an intensity of 1.9 kV using a 2510 electroporator (Eppendorf).

Immediately afterwards, the cells were resuspended with 450 mL of liquid LB medium, and the contents were then placed in a 2.0 mL microtube. This microtube was kept at 37 °C, with constant agitation at 150 rpm, for a period of 1 hour. Then, 100 µL of the transform were distributed onto a culture plate containing LB agar medium, to which 100 µg / mL of ampicillin and 25 µg / mL of chloramphenicol were added. These plates were incubated at 37 °C for a total of 16 hours.

Subsequently, randomly selected colonies from the plates were pre-inoculated separately into test tubes, each containing 3 mL of liquid LB medium, and containing ampicillin (100 µg / mL) and chloramphenicol (25 µg / mL) as selection antibiotics. After a 16-hour incubation period under shaking at 37 °C, the culture was transferred to 100 mL of liquid LB medium containing the selection antibiotics. The temperature was maintained at 37 °C on the shaker until the optical density (OD) reached 0.6, measured by spectrophotometer at 600 nm.

At this point, 15 mL of the bacterial culture were distributed into six conical tubes, with the final concentrations of 0.1 mM and 1 mM of IPTG (isopropyl-β-D-thiogalactopyranose), and a final concentration of 10 g / L of lactose, respectively, added to each tube.

Induction with IPTG and lactose was performed under the following conditions: 3 hours and 16 hours; at 37 °C, 22 °C, and 11 °C. Immediately afterwards, centrifugation was performed at 3500 x g for 15 minutes at 4 °C. The bacterial pellets were stored in 50 mL conical tubes and kept at -20 °C, awaiting the subsequent sonication step. This procedure consisted of resuspending the bacterial pellets in MCAC-0 buffer (20 mM Tris with pH 7.9, 0.5 M NaCl, 10 % glycerol, and 1 mM PMSF), followed by sonication using a procedure already standardized by our team. The sonicated material was centrifuged at 10,000 x g, 4 °C, for 15 minutes. Following the sonication procedure, analysis was performed by sodium dodecyl sulfate-containing polyacrylamide gel electrophoresis (SDS-PAGE) and Western blot analysis.

### Example 14 - Transformation and Expression of the EP1 Protein in E. coli Arctic Xpress DE3

The transformation of *Escherichia coli* Arctic Xpress DE3 bacteria with pRSET plasmids containing the EP1 gene was performed following this procedure:

First, 1 µL (corresponding to 50 ng) of the plasmid was added to a total of 50 µL of competent *E. coli* Arctic Xpress DE3 cells. Subsequently, this mixture was exposed to an electrical pulse with an intensity of 1.9 kV using a 2510 electroporator (Eppendorf).

Immediately afterwards, the cells were resuspended with 450 mL of liquid LB medium, and the contents were then placed in a 2.0 mL microtube. This microtube was kept at 37 °C, with constant agitation at 150 rpm, for a period of 1 hour. Then, 100 µL of the transform were distributed onto culture plates containing LB agar medium, to which 100 µg / mL of ampicillin were added. These plates were incubated at 37 °C for a total of 16 hours.

Subsequently, randomly selected colonies from the plates were pre-inoculated separately into test tubes, each containing 3 mL of liquid LB medium containing ampicillin as the selection antibiotic. After a 16-hour incubation period under shaking at 37 °C, the culture was transferred to 50 mL of liquid LB medium containing the selection antibiotic. The temperature was maintained at 37 °C on the shaker until the optical density (OD) reached 0.6, measured by spectrophotometer at 600 nm.

At this point, the bacterial culture was distributed into six conical tubes, with the final concentrations of 0.1 mM and 1 mM of IPTG (isopropyl-β-D-thiogalactopyranose), and lactose (10 g / L, final concentration), respectively, added to each tube.

Induction with IPTG and lactose was performed under the following conditions: 3 hours and 16 hours at 11 °C. Immediately afterwards, centrifugation was performed at 4000 rpm for 15 minutes at 4 °C. The bacterial pellets were stored in 50 mL conical tubes at -20 °C, awaiting the subsequent sonication step. This procedure consisted of resuspending the bacterial pellets in MCAC-O buffer (20 mM Tris with pH 7.9, 0.5 M NaCl, 10 % glycerol and 1 mM PMSF), followed by sonication using a procedure already standardized by our team. The sonicated material was centrifuged at 14,000 rpm, 4 °C, for 15 minutes. Following the sonication procedure, analysis was performed by sodium dodecyl sulfate-containing polyacrylamide gel electrophoresis (SDS-PAGE) and Western blot analysis.

### Example 15 - Transformation and Expression of the EP1 Protein in E. coli Tuner DE3

The transformation of *Escherichia coli* Turner DE3 bacteria with pRSET plasmids containing the EP1 gene was performed following this procedure:

First, 1 µL (corresponding to 50 ng) of the plasmid was added to a total of 50 µL of competent *E. coli* Tuner DE3 cell. Subsequently, this mixture was exposed to an electrical pulse with an intensity of 1.9 kV using a 2510 electroporator (Eppendorf).

Immediately afterwards, the cells were resuspended with 450 mL of liquid LB medium, and the contents were then placed in a 2.0 mL microtube. This microtube was kept at 37 °C, with constant agitation at 150 rpm, for a period of 1 hour. Then, 100 µL of the transform were distributed onto culture plates containing LB agar medium, to which 100 µg / mL of ampicillin were added. These plates were incubated at 37 °C for a total of 16 hours.

Next, randomly selected colonies from the plates were pre-inoculated separately into test tubes, each containing 3 mL of liquid LB medium containing ampicillin as the selection antibiotic. After a 16-hour incubation period under shaking at 37 °C, the culture was transferred to 50 mL of liquid LB medium containing the selection antibiotic. The temperature was maintained at 37 °C on the shaker until the optical density (OD) reached 0.6, measured by spectrophotometer at 600 nm.

At this point, the bacterial culture was distributed into two conical tubes, to which IPTG (isopropyl-β-D-thiogalactopyranose) was added at a final concentration of 0.1 mM and 1.0 mM, and lactose (10 g / L, final concentration), respectively, in each tube.

Induction with IPTG and lactose was performed under the following conditions: 3 hours and 16 hours; at 37 °C and 22 °C. Immediately afterwards, centrifugation was performed at 4000 rpm for 15 minutes at 4 °C. The bacterial pellets were stored in 50 mL conical tubes and kept at -20 °C, awaiting the subsequent sonication step. This procedure consisted of resuspending the bacterial pellets in MCAC-O buffer (20 mM Tris with pH 7.9, 0.5 M NaCl, 10 % glycerol and 1 mM PMSF), followed by sonication using a procedure already standardized by our team. The sonicated material was centrifuged at 14,000 rpm, 4 °C, for 15 minutes. Following the sonication procedure, analysis was performed by sodium dodecyl sulfate-containing polyacrylamide gel electrophoresis (SDS-PAGE) and *Western blot* analysis.

### Example 17 - Purification of recombinant EP1 and EP2 proteins obtained in Examples 13 to 16

The proteins were purified using immobilized metal affinity chromatography (IMAC). For this purpose, a purification column containing Ni²⁺ ions (QIAGEN) was used, following the manufacturer's instructions (QIAGEN). Protein elution was performed using an imidazole concentration curve from 100 mM to 500 mM. Subsequently, the samples were quantified using Bradford reagent (Bio-rad), following the manufacturer's instructions. The yield was determined from the sum of the concentrations in milligrams of protein obtained in each elution of the isolated proteins in parallel with the volume of bacterial growth initially used. Therefore, if 10 mg and 5 mg of proteins were obtained in 1 L of induction, the estimated yields are 10 mg / L and 5 mg / L, respectively.

### Example 18 - Analysis by polyacrylamide gel electrophoresis and Western blot of the recombinant proteins obtained in Examples 13 to 16

The expression of recombinant proteins was evaluated using polyacrylamide gel electrophoresis (SDS-PAGE). Electrophoresis was performed in tris-glycine buffer for 1 hour and 30 minutes at 150 V. The molecular weight marker "Blueye Prestained Protein Marker" (Sigma Aldrich) was used as a reference. Samples were resuspended in resuspension buffer (200 mM Tris-HCl pH 6.8, 0.1 % bromophenol blue, 4 % SDS, 4 % β-mercaptoethanol, and 20 % glycerol). Subsequently, the samples were denatured at 95 °C for 10 minutes and then applied to the gels. Gel staining for visualization of protein bands was performed using a commercial buffer from Scienco.

For Western blot assays, membranes were prepared using an SDS-PAGE gel with the samples (unstained) and a semi-dry system (Bio-rad) in tris-glycine-SDS buffer. The system was subjected to a constant voltage of 10 V for 1 hour and 10 minutes. After being removed from the chamber, the membrane was stored in 1X PBS at 4 °C until development. Next, the development was performed where the membrane was first blocked for 30 minutes using 1X PBS solution with 3 % BSA, after which the membrane was washed with MilliQ water for 5 minutes and then the anti-6xhisG monoclonal antibody (Invitrogen), diluted to a concentration of 1:3000, was added for 1 hour. The membrane was then washed 3 times, for 5 minutes each time, with 1X PBS solution supplemented with 0.05 % Tween 20. Mouse anti-IgG secondary antibody + alkaline phosphatase (Seracare), diluted 1:10000, was added and incubated for 1 hour. The membrane was then washed again with PBS Tween, as previously described, and water. The reaction was then revealed using BCIP / NBT buffer (Invitrogen) for up to 10 minutes, followed by photographic documentation.

### References

ALAPE-GIRÓN, A.; FLORES-DÍAZ, M.; GUILLOUARD, I.; NAYLOR, C.E.; TITBALL, R.W.; RUCAVADO, A.; THELESTAM, M. Identification of residues critical for toxicity in Clostridium perfringens phospholipase C, the key toxin in gas gangrene. European Journal of Biochemistry, v. 267, n. 16, p. 5191-5197, 2000.
AQUINO, P. L. M., FONSECA, F. S., MOZZER, O. D., GIORDANO, R. C., JR, R. SOUSA. Optimization of the Production of Inactivated Clostridium novyi Type B Vaccine Using Computational Intelligence Techniques. Appl. Biochem. Biotechnol. 179:895-909, 2016.
ASSIS, R. A., LOBATO, F. C. F., MARTINS, N. E., NASCIMENTO, R. A. P., ABREU, V. L. V., UZAL, F. A. An outbreak of malignant edema in cattle. Revista Portuguesa de Ciências Veterinárias. 97:143-145, 2002.
BARUAH N, AHAMAD N, MAITI S, HOWLADER DR, BHAUMIK U, PATIL VV, CHAKRABARTI MK, KOLEY H, KATTI DS. Development of a Self-Adjuvanting, Cross-Protective, Stable Intranasal Recombinant Vaccine for Shigellosis. ACS Infect Dis. 2021 Dec 10;7(12):3182-3196. Doi: 10.1021/acsinfecdis.1c00345. Epub 2021 Nov 4. PMID: 34734708.
BERGMEYER, H. U., HERDER, M., REF, R. International federation of clinical chemistry (IFCC), J. Clin. Chem. Clin. Biochem. 24:497-510, 1986.
BRANDI, I. V., MOZZER, O. D., VANDER JORGE, E., PASSOS, F. J. V., PASSOS, F. M. L., CANGUSSU, A. S. R., SOBRINHO, E. M. Growth conditions of clostridium perfringens type B for production of toxins used to obtain veterinary vaccines. Bioprocess Biosyst. Eng. 37:1737-1742, 2014.
BRANDI, I. V., SANTOS, E. M., CARVALHO, B. M., DURÃES, C. A., FARIAS, P. K., SARI, R. S., CANGUSSU, A. S. R., PESSOA, A. J. Total combining power: Technique for the evaluation of the quality control process of clostridiosis vaccines. J Microbiol Methods. 130:164-168, 2016.
BRITO, H. S., ALENCAR, F. C., ALBURQUERQUE, B., SILVA, M. G., FÉLIX, M. K., MULHOLLAND, D. S., CANGUSSU, A. S. Pathologic findings on ruminant enteric clostridial diseases reveal specificities and differences among iota and iota-like toxins. Revista de Ciências Agrícolas. 38(2):157-174, 2021.
CANGUSSU, A. S. R., MARIÚBA, L. A. M., LALWANI, P., PEREIRA, K. D. E., ASTOLPHI-FILHO, S., ORLANDI, P. P., NOGUEIRA, P. A. A hybrid protein containing MSP1a repeats and Omp7, Omp8 and Omp9 epitopes protect immunized BALB/c mice against anaplasmosis. Veterinary research. 49(1):1-11, 2018.
CARDOSO, V. M., PAREDES, S. A., CAMPANI, G., GONÇALVES, V. M., ZANGIROLAMI, T. C. ClearColi as a platform for untagged pneumococcal surface protein A production: cultivation strategy, bioreactor culture, and purification. Applied microbiology and biotechnology. 1-19, 2022.
CARVALHO, T. B., ZEN, S. A cadeia de Pecuária de Corte no Brasil: evolução e tendências. Rev. iPecege. 3:85-99, 2017.
CORREA, A.; OPPEZZO, P. Tuning different expression parameters to achieve soluble recombinant proteins in E. coli: Advantages of high-throughput screening. Biotechnology Journal. 6:715-730, 2011.
DUARTE, L. S., MATTE, C. R., DALL CORTIVO, P. R., NUNES, J. E. S., BARSÉ, L. Q., BIZARRO, C. V., AYUB, M. A. Z. Expression of Bacillus amyloliquefaciens transglutaminase in recombinant E. coli under the control of a bicistronic plasmid system in DO-stat fed-batch bioreactor cultivations. Brazilian Journal of Microbiology. 52(3):1225-1233, 2021.
Embrapa, 2001. Clostridiosis em Bovinos. Link: https://www.embrapa.br/documents/1354377/1743404/Clostridio sisVarzeaGrande10-11.pdf/66d4a459-5b70-4879-b79d-909949cc9032?version=1.0
FARIAS, L. D. AVILA; AZEVEDO, M. S.; TROST, M. E.; DE LA CÔRTE, F. D.; IRIGOYEN, L. F.; VARGAS, A. C. Acute myonecrosis in horse caused by Clostridium novyi type A. Brazilian Journal of Microbiology. 45:221-224, 2014.
FÉLIX, M. K. C., DEUSDARÁ, T. T., SANTOS, L. S. S., AGUIAR, R. W. S., CORRÊA, R. F. T., BRANDI, I. V., CANGUSSU, A. S. R. Inactivated alpha toxin from Clostridium novyi type B in nano-emulsion protect partially protects Swiss mice from lethal alpha toxin challenge. Scientific reports. 9(1):1-9, 2019.
IBGE, 2021. Rebanho de Bovinos (Bois and Vacas). Link: https://www.ibge.gov.br/explica/producao-agropecuaria/bovinos/br
FÉLIX, M. K., LEITE, E. R., DUTRA, L. R., RIBEIRO, M. A., MOURA, W. S., FERREIRA, T. P., CANGUSSU, A. S. Secondary metabolites produced by solid-state fermentation of a neotropical Aspergillus flavus strain confer anti-mosquito activity with long shelf-life. Industrial Crops and Products. 180:114743, 2022.
FERRAZ, J. B. S., FELÍCIO, P. E. Production systems - An example from Brazil. Meat Science. 84:238-243, 2010.
FIELD, J. K., EUERBY, M. R., HASELMANN, K. F., PETERSSON, P. Investigation into reversed-phase chromatography peptide separation systems Part IV: Characterisation of mobile phase selectivity differences. Journal of Chromatography A. 1641:461986, 2021.
FOUAD, E. A., TOALEB, N. I., HASSAN, S. E., EL SHANAWANY, E. E., KESHTA, H. G., ABDEL-RAHMAN, E. H., HEGAZI, A. G. RESEARCH ARTICLE Evaluation of the therapeutic effect of propolis on Fasciola gigantica and Clostridium novyi type B infections in sheep. Tropical Biomedicine. 38(2):102-110, 2021.
LOBATO FCF, SALVARANI FM, GONÇALVES LA, PIRES PS, SILVA ROS, ALV GG, NEVES M, JÚNIOR CA DE O, PEREIRA PLL. Clostridiosis dos animais de produção. Veterinária e Zootecnia. 20:29-48, 2023.
GRUN, J. L., MAURER, P. H. Different T helper cell subsets elicited in mice utilizing two different adjuvant vehicles: the role of endogenous interleukin 1 in proliferative responses. Cell. Immunol. 121:134-145, 1989.
JONASSON, P.; LILJEQVIST, S.; NYGREN, P. A.; STAHL, S. Genetic design for facilitated production and recovery of recombinant proteins in Escherichia coli. Biotechnol. Appl. Biochem. 35:91-105, 2002.
KHORASANI, A., MADADGAR, O., SOLEIMANJAHI, H., KEYVANFAR, H., MAHRAVANI, H. Evaluation of the efficacy of a new oil-based adjuvant ISA 61 VG FMD vaccine as a potential vaccine for cattle. Iranian Journal of Veterinary Research. 17:8-12, 2016.
KOJIMA, K., SUDO, Y. Expression of microbial rhodopsins in Escherichia coli and their extraction and purification using styrene-maleic acid copolymers. STAR protocols. 3(1):101046, 2022.
LE GRATIET, T., LE MARECHAL, C., DEVAERE, M., CHEMALY, M., WOUDSTRA, C. Exploration of the Diversity of Clustered Regularly Interspaced Short Palindromic Repeats-Cas Systems in Clostridium novyi sensu lato. Frontiers in Microbiology. 2577, 2021.
LECLERCQ, S. Y., SANTOS, R. M. M., MACEDO, L. B., CAMPOS, P. C., FERREIRA, T. C., ALMEIDA, J. G., SENIUK, J. G. T., SERAKIDES, R., SILVA-CUNHA, A., FIALHO, S. L. Evaluation of water-in-oil-in-water multiple emulsion and microemulsion as potential adjuvants for immunization with rabies antigen. European Journal of Pharmaceutical Sciences. 43:378-385, 2011.
LEE, S. Y.; CHOI, J. H.; XU, Z. Microbial cell-surface display. Trends Biotechnol. 21(1):45-52, 2003.
LIMA, C. G. R. D., LOBATO, Z. I. P., PIRES, P. S., SILVA, R. O. S., SALVARANI, F. M., ASSIS, R. A., LOBATO, F. C. F. Padronização de teste de potência in vitro para vacinas que contenham toxoide alfa de Clostridium novyi tipo B. Arq. Inst. Biol. 78:507-512, 2011.
LIMA, C.G.R.D.; LOBATO, Z.I.P.; PIRES, P.S.; SILVA, R.O.S.; SALVARANI, F.M.; ASSIS, R.A.; LOBATO, F.C.F. Padronização de teste de potência in vitro para vacinas que contenham toxoide alfa de Clostridium novyi tipo B. Arquivos do Instituto Biológico, v. 78, p. 507-512, 2020.
LIU, J.; KOTHE, M.; ZHANG, J.; OLOO, E.; STEGALKINA, S.; MUNDLE, S.T.; ANDERSON, S.F. Novel structural insights for a pair of monoclonal antibodies recognizing nonoverlapping epitopes of the glucosyltransferase domain of Clostridium difficile toxin B. Current Research in Structural Biology, v. 4, p. 96-105, 2022.
LOBATO, F.C.F., DIAS, L.D., SALVARANI, F.M., MARTINS, N.E., NASCIMENTO, R.A.O., ASSIS, R.A. Avaliação da potência de vacinas contra Clostridium septicum comercializadas no Brasil. Arq. Inst. Biol. 75, 225-228, 2008.
LOBATO, F.C.F.; DIAS, L.D.; SALVARANI, F.M.; MARTINS, N.É.; DO NASCIMENTO, R.A.P.; DE ASSIS, R.A. Avaliação da potência de vacinas contra Clostridium septicum comercializadas no Brasil. Arquivos do Instituto Biológico, v. 75, p. 225-228, 2021.
LOBATO, F.C.F.; SALVARANI, F.M.; GONÇALVES, L.A.; PIRES, P.S.; SILVA, R.O.S.; ALVES, G.G.; NEVES, M.; JÚNIOR, C.A.O.; PEREIRA, P.L.L. Clostridiosis Dos Animais De Produção. Veterinária e Zootecnia, v. 20, p. 29-48, 2013.
MA, M., BOYD, J.T., TRINH, H.T., COOMBS, J.W., FERMANN, G.J. Fatal myocarditis due to Clostridium novyi type B in a previously healthy woman: Case report and literature review. Scand. J. Infect. Dis. 39, 77-80, 2007.
MOREIRA, G. M. S. G., SALVARANI, F. M., DA CUNHA, C. E. P., MENDONÇA, M., MOREIRA, Â. N., GONÇALVES, L. A., CONCEIÇÃO, F. R. Immunogenicity of a trivalent recombinant vaccine against Clostridium perfringens alpha, beta, and epsilon toxins in farm ruminants. Scientific reports. 6(1):1-9, 2016.
NAGAHAMA, M.; ODA, M.; KOBAYASHI, K.; OCHI, S.; TAKAGISHI, T.; SHIBUTANI, M.; SAKURAI, J. A recombinant carboxy-terminal domain of alpha-toxin protects mice against Clostridium perfringens. Microbiology and immunology, v. 57, n. 5, p. 340-345, 2013.
NAGAHAMA, M.; OTSUKA, A.; SAKURAI, J. Role of tyrosine-57 and -65 in membrane-damaging and sphingomyelinase activities of Clostridium perfringens alpha-toxin. Biochimica et Biophysica Acta (BBA)-Molecular Basis of Disease, v. 1762, n. 1, p. 110-114, 2006.
NASCIMENTO, R.A.P. LOBATO, F.C.F, ABREU, V.L.V., MARTINS, N.E., ASSIS, R.A., CARVALHO FILHO, M.B. Avaliação de vacinas contra Clostridium novyi tipo B. Arq. Bras. Med. Vet. Zootec. 56, 1-6, 2004.
NAVARRO, M.A.; DUTRA, F.; BRIANO, C.; ROMERO, A.; PERSIANI, M.; FREEDMAN, J.C.; MORRELL, E.; BEINGESSER, J.; UZAL, F.A. Pathology of Naturally Occurring Bacillary Hemoglobinuria in Cattle. Veterinary pathology, v. 54, n. 3, p. 457-466, 2017.
NOSHAHRI, N.G.; NAJAFI, M.F.; KAKHKI, A.M.; MAJIDI, B.; MEHRVARZ, M. Identification and cloning of highly epitopic regions of Clostridium novyi alpha toxin. Turkish Journal of Biology, v. 40, n. 6, p. 1219-1226, 2016.
ODA, M.; TERAO, Y.; SAKURAI, J.; NAGAHAMA, M. Membrane-binding mechanism of Clostridium perfringens alpha-toxin. Toxins, v. 7, n. 12, p. 5268-5275, 2015.
PARK, S. S.; LILLEHOJ, H. S.; ALLEN, P. C.; PARK, D. W.; FITZCOY, S.; BAUTISTA, D. A.; LILLEHOJ, E. P. Immunopathology and Cytokine Responses in Broiler Chickens Coinfected with Eimeria maxima and Clostridium perfringens with the Use of an Animal Model of Necrotic Enteritis. Avian Diseases. 52:14-22, 2014.
PEREZ-CASAL J, PRYSLIAK T, MAINA T, SULEMAN M, JIMBO S. Status of the development of a vaccine against Mycoplasma bovis. Vaccine. 2017 May 19;35(22):2902-2907. doi: 10.1016/j.vaccine.2017.03.095. Epub 2017 Apr 19. PMID: 28433326.
RADOSTITS O.M., GAY C.C., BLOOD D.C. & HINCHCLIFF K.W. Clínica Veterinária: um tratado de doenças dos bovinos, ovinos, suínos, caprinos and eqüinos. Guanabara Koogan. 347-360, 2002.
SANTOS, E. M. S., ALMEIDA, A. C., SANTOS, H. O., CANGUSSU, A. R., COSTA, K. S., ALVES, J. N., AGUIAR, R. W. S. Mechanism of Brassica oleracea performance in bovine infectious mastitis by bioinformatic analysis. Microbial pathogenesis. 129:19-29, 2019.
SANTOS PS, NASCIMENTO R, RODRIGUES LP, SANTOS FAA, FARIA PCB, ET AL. Functional Epitope Core Motif of the Anaplasma marginale Major Surface Protein 1a and Its Incorporation onto Bioelectrodes for Antibody Detection. Plosone. 7(3):33045, 2012.
SILVESTRE, B. T., RABELO, É. M., VERSIANI, A. F., DA FONSECA, F. G., SILVEIRA, J. A., BUENO, L. L., RIBEIRO, M. F. Evaluation of humoral and cellular immune response of BALB/c mice immunized with a recombinant fragment of MSP1a from Anaplasma marginale using carbon nanotubes as a carrier molecule. Vaccine. 32(19):2160-2166, 2014.
SKARIN, H., SEGERMAN, B. Plasmidome Interchange between Clostridium botulinum, Clostridium novyi, and Clostridium haemolyticum Converts Strains of Independent Lineages into Distinctly Different Pathogens. Plosone. 9:107777, 2014.
SOBRINHO, E. M., CANGUSSU, A. S., BRANDI, I. V., SARI, R. S., ALMEIDA, A. C., COLEN, F., SANTOS, H. O. Modified toxin-binding inhibition (ToBI) test for epsilon antitoxin determination in serum of immunized rabbits. Veterinary Immunology and Immunopathology. 138(1-2):129-133, 2010.
SUN, H.W, WEI, C., LIU,B.S., JING,H.M., FENG, Q., TONG, Y.N., YANG, Y., YANG, L.Y., ZUO,Q.F., ZHANG, Y., ZOU, Q.M., ZENG, H. Induction of systemic and mucosal immunity against methicillin-resistant Staphylococcus aureus infection by a novel nanoemulsion adjuvant vaccine. Int. J. Nanomedicine. 10:7275-90, 2015.
TEHRANI, N.K., MAHDAVI, M., MALEKI, F., ZARRATI, S., TABATABAIE, F. The role of Montanide ISA 70 as an adjuvant in immune responses against Leishmania major induced by thiol-specific antioxidant-based protein vaccine. J. Parasit. Dis. 40:760-767, 2016.
TSUTSUI, K.; MINAMI, J.; MATSUSHITA, O.; KATAYAMA, S.; TANIGUCHI, Y.; NAKAMURA, S.; OKABE, A. Phylogenetic analysis of phospholipase C genes from Clostridium perfringens types A to and Clostridium novyi. Journal of bacteriology, v. 177, n. 24, p. 7164-7170, 1995.
UZAL, F. A., PARAMIDANI, M., ASSIS, R., MORRIS, W., MIYAKAWA, M. F. Outbreak of clostridial myocarditis in calves. Vet. Rec. 152:134-136, 2003.
UZAL, F.A.; VIDAL, J.E.; MCCLANE, B.A.; GURJAR, A.A. Clostridium perfringens toxins involved in mammalian veterinary diseases. The open toxinology journal, v. 2, p. 24, 2010.
VEENSTRA, K.A., WANG, T., ALNABULSI, A., DOUGLAS, A., RUSSELL, K.S., TUBBS, L., AROUS, J.B., SECOMBES, C.J. Analysis of adipose tissue immune gene expression after vaccination of rainbow trout with adjuvanted bacterins reveals a combination with side effects. Mol. Immunol. 88:89-98, 2017.
WALKER, D. J. F., HEAP, J. T., WINZER, K., MINTON, N. P. A genetic assay for gene essentiality in Clostridium. Anaerobe. 42:40-43, 2016.
WALKER, N.; HOLLEY, J.; NAYLOR, C.E.; FLORES-DIAZ, M.; ALAPE-GIRÓN, A.; CARTER, G.; TITBALL, R.W. Identification of residues in the carboxy-terminal domain of Clostridium perfringens α-Toxin (Phospholipase C) which are required for its biological activities. Archives of Biochemistry and Biophysics, v. 384, n. 1, p. 24-30, 2000.
ZENG, J., DENG, G., WANG, J., ZHOU, J., LIU, X., XIE, Q., WANG, Y. Potential protective immunogenicity of recombinant Clostridium perfringens α-β2-β1 fusion toxin in mice, sows, and cows. Vaccine. 29(33):5459-5466, 2011.

## Claims

1. Recombinant nucleic acid molecule **characterized by** the fact that it comprises the nucleic acid sequences as defined by SEQ ID NO: 1 or by SEQ ID NO: 3 and its degenerated sequences encoding the same polypeptidic sequence.

2. Epitope of recombinant *alpha toxin from C. novyi* **characterized by** the fact that it comprises the peptide sequences as defined by SEQ ID NO: 2 or by SEQ ID NO: 4.

3. Epitope, according to claim 2, **characterized by** the fact that it further comprises a poly histidine tail (6xHIS) at the amino-terminal end.

4. Expression vector **characterized by** the fact that it comprises the nucleic acid molecules as defined in claim 1 and their regulatory regions.

5. Expression vector, according to claim 4, **characterized by** the fact that the regulatory regions are selected from BamHI and EcoRI.

6. Vaccine composition **characterized by** the fact that it comprises one or more epitopes as defined in claim 2 and veterinary acceptable excipients.

7. Vaccine composition **characterized by** the fact that it comprises both epitopes as defined in claim 2 and veterinary acceptable excipients.

8. Vaccine composition, according to claim 6, **characterized by** the fact that it is a water / oil emulsion.

9. Vaccine composition, according to claim 6, **characterized by** the fact that it is for the prevention or treatment of clostridiosis in a subject suffering therefrom.

10. Use of one or more epitopes as defined in claim 2, **characterized by** the fact that it is in the preparation of a vaccine composition or preventing or treating clostridiosis in a subject suffering therefrom.

11. Method for preventing or treating clostridiosis in a subject suffering therefrom **characterized by** the fact that it comprises administering the vaccine composition as defined in claim 6.

12. Method for producing a vaccine composition against clostridiosis **characterized by** the fact that it comprises the following steps:
i) Transforming *E. coli* cells with an expression plasmid comprising the molecules as defined in claim 1;
ii) Culturing said cells in bioreactors via the fed-batch process;
iii) Purifying the product of the culturing step ii);
iv) Preparing the vaccine composition comprising the epitopes obtained after step iii).
